(19) Europäisches Patentamt
European Patent Office
Office européen des brevets

(11) EP 2 581 359 B1

(12) # EUROPEAN PATENT SPECIFICATION

(45) Date of publication and mention of the grant of the patent:
**30.03.2016 Bulletin 2016/13**

(21) Application number: **11791921.7**

(22) Date of filing: **07.06.2011**

(51) Int Cl.:
***C07C 17/093*** (2006.01) ***C07C 19/07*** (2006.01)
***B01D 35/143*** (2006.01) ***G21F 9/02*** (2006.01)
***G01M 3/20*** (2006.01) ***G21C 17/00*** (2006.01)
***G21D 3/00*** (2006.01) ***G21G 4/06*** (2006.01)
***G21G 1/00*** (2006.01)

(86) International application number:
**PCT/CN2011/075377**

(87) International publication number:
**WO 2011/153930 (15.12.2011 Gazette 2011/50)**

(54) **REAGENT FOR TESTING PURIFICATION CAPACITY OF RADIOACTIVE GAS IN A NUCLEAR POWER PLANT, PREPARATION METHOD THEREOF AND IODIDE FILTER TESTING EQUIPMENT USING THIS REAGENT**

REAGENS ZUM TESTEN DER REINIGUNGSKAPAZITÄT EINES RADIOAKTIVEN GASES IN EINEM KERNKRAFTWERK, HERSTELLUNGSVERFAHREN DAFÜR UND JOD-FILTERTESTAUSRÜSTUNG MIT DIESEM REAGENS

RÉACTIF POUR TESTER LA CAPACITÉ DE PURIFICATION DE GAZ RADIOACTIF DANS UNE CENTRALE NUCLÉAIRE, PROCÉDÉ DE PRÉPARATION ASSOCIÉ ET ÉQUIPEMENT D'ESSAI DE FILTRE D'IODE UTILISANT CE RÉACTIF

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.08.2010 CN 201020514964 U**
**07.06.2010 CN 201010193321**

(43) Date of publication of application:
**17.04.2013 Bulletin 2013/16**

(73) Proprietors:
- **China General Nuclear Power Corporation Shenzhen, Guangdong (CN)**
- **Dayabay Nuclear Power Operations and Management Co., Ltd. Shenzhen, Guangdong 518031 (CN)**
- **China Institute For Radiation Protection Shanxi 030006 (CN)**

(72) Inventors:
- **DU, Jianxing Guangdong 518031 (CN)**
- **ZHANG, Dayong Guangdong 518031 (CN)**
- **BIAN, Shouli Guangdong 518031 (CN)**
- **LIU, Daohe Guangdong 518031 (CN)**
- **WU, Yukun Guangdong 518031 (CN)**
- **LI, Xianfeng Guangdong 518031 (CN)**
- **YANG, Lietang Guangdong518031 (CN)**
- **KONG, Haixia Shanxi 030006 (CN)**
- **QIU, Dangui Shanxi 030006 (CN)**
- **SHI, Yingxia Shanxi 030006 (CN)**
- **GUO, Liangtian Shanxi 030006 (CN)**
- **WU, Zhenlong Shanxi 030006 (CN)**
- **HAN, Lihong Shanxi 030006 (CN)**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 581 359 B1

(74) Representative: **Bosch, Matthias et al**
**Bosch Jehle**
**Patentanwaltsgesellschaft mbH**
**Flüggenstrasse 13**
**80639 München (DE)**

(56) References cited:
**CN-A- 101 875 597** **CN-U- 201 791 424**
**CN-Y- 2 329 968** **FR-A1- 2 553 562**
**US-A- 4 016 242** **US-A1- 2006 177 944**

- **MEI YING.: 'Radioactive methyl-iodide method for on-site test of iodine adsorbers.' NUCLEAR TECHNIQUES vol. 31, no. 4, April 2008, pages 289 - 292, XP008169076**

## Description

FIELD OF THE INVENTION

This invention belongs to the technical field of advanced pressurized water reactor nuclear power of million-kilowatt, and relates to supplies for detecting the safety of nuclear power, preparation method thereof and an equipment using the same. More particularly, this invention relates to a reagent for testing the purification capacity of radioactive gas in nuclear power plant, preparation method thereof and an iodide filter testing equipment using this reagent.

BACKGROUND OF THE INVENTION

In the radioactive reactor fission products of an advanced pressurized water reactor nuclear power plant of million-kilowatt, gaseous radioactive iodine mainly exists as the molecular iodine ($^{129}I_2$, $^{131}I_2$) and the organic iodine ($CH_3{}^{131}I$). Wherein the elemental molecular iodine accounts for 90~95% of the gaseous radioactive iodine, and the organic iodine only accounts for 5~10% of the gaseous radioactive iodine. Although the concentration of radioactive iodine is low, for example, the concentration of radioactive iodine in the air within a reactor containment usually does not exceed 1 Ci/m$^3$ or 8$\mu$g/M$^3$ after an accident of design basis, the released radioactive iodine is still very harmful to human health since human thyroid has a very high absorption capacity of the radioactive iodine. Therefore, in an exhaust system, an iodide filter (or called as an iodine absorber) is used to filter the iodine and intercept the radioactive iodine possibly existing in the air, in which case a safe, harmless and fresh air is provided to the service area, or the radioactive iodine in the emission of radioactive manufacturability exhaust is filtered so as to reduce the radioactive iodine and control the emission of radioactive iodine to the atmosphere thereby reducing the impact on the environment.

To ensure the availability of iodide filter in some important ventilation systems of the nuclear power plant, it is needed to carry out a field efficiency test on the iodide filter regularly, aiming to make its interception ability of radioactive iodine meet the corresponding requirement. At present, in the domestic nuclear power plant, AFNOR NF MG2-206 ⟨⟨Test Method for Purification Coefficient of Iodide Filter⟩⟩ developed by French Mechanical Standard Committee is applied to the field efficiency test ("radioactive methyl iodide method") on the iodide filter. In this method, a methyl iodide gas generator is used to put some radioactive methyl iodide tracers in the upstream of the iodide filter, a sampling carbon box is used for sampling in the upstream and downstream of the iodide filter, and then a $\gamma$ spectrometer is utilized for analyzing the radioactive activity of the carbon box in both upstream and downstream. After that, the filtering characteristic of the iodide filter is determined by utilization of a ration of the radioactive activity (purification coefficient) in both upstream and downstream.

The existing radioactive methyl iodide gas tracer is prepared by the reaction between radioactive and non-radioactive sodium iodide solutions and a dimethyl sulfate. the specific equation is as follows:

$$Na^{127}I + Na^{131}I + (CH_3)_2SO_4 = Na_2SO_4 + CH_3{}^{131}I + CH_3{}^{127}I$$

Radioactive methyl iodide is airborne iodide, and its physicochemical properties are similar to those of other organic iodides. The radioactive methyl iodide with representative characteristic also has the following advantages: low boiling point, volatile, easy to produce, easy to measure, difficult to deposition, long desorption time and convenient for measurement. The radioactive methyl iodide belongs to one kind of iodide which is the most difficult to be absorbed by the iodide filter and cause no influence on the filtering characteristics of the iodide filter. Moreover, its half-life is short, and its radiation effects on the human and the environment are also small. For these reasons, the radioactive methyl iodide is used in the field test on the iodide filter. However, since the material dimethyl sulfate in the radioactive methyl iodide method is highly toxic, of which the toxicity is similar to that of mustard gas, the safety and environmental protection requirements on its storage and usage are greatly high, which leads to a high risk during the test. Although the storage and usage amount of the dimethyl sulfate is very low in the nuclear power plant, it is still needed to make a registration and record in the corresponding safety supervision departments and public security departments; this is because the dimethyl sulfate used in the nuclear power plant is a highly toxic product which belongs to one kind of chemical reagent under the state control. Meanwhile, it is also required to establish a storage warehouse and corresponding management system in the nuclear power plant, which should comply with the correlated regulations. In this circumstance, the nuclear power plant has been paying a large amount of manpower and financial resources for ensuring its absolute safety, while still there are large risks in management.

The following problems exist in the existing methyl iodide generator: 1, the conventional equipment is provided with an iodine remover at the air inlet, wherein the radioactive gas flowing back to an intake pipe road is removed through the iodine remover in the event of a device failure, so that the radioactive gas is avoided to be discharged out of the methyl iodide generator to prevent the tester from pollution. However, the iodine remover mainly acts upon the radioactive gas with an iodine removing effect, while it cannot act upon on the radioactive liquid. Hence, a failure occurs if some

radioactive liquids enter. 2, in the existing equipment, the internal needle and the external needle of a sleeve needle are all with a flat head. In this case, if there are impurities in the reaction flash or solid product is generated during reaction together with the liquid agitation during reaction, it would be very easy to form a blockage between the internal needle and the external needle with a flat head. 3, when a blockage occurs (such as the needle or a throttle valve is blocked) below the air entry of an injection pump in the generation loop, the existing methyl iodide generator cannot stop generating the methyl iodide automatically. The generated methyl iodide gas cannot be discharged due to the blockage, while a positive pressure occurs in the reaction flask with the increase of methyl iodide gas. In this case, the liquid iodine source in a small bottle is pressured into the intake pipe and further lost, or the methyl iodide gas spills into the generator from the external needle and a bottle stopper. Even if the failure is overcome, the remaining iodine source cannot be used in tests due to its loss, which is further wasted thereafter.

US 4 016 242 A describes radioactive iodine and radioactive methyliodide that can be more than 99.7 per cent removed from the air stream of a nuclear reactor by passing the air stream through a 2-inch thick filter which is made up of impregnated charcoal prepared by contacting the charcoal with a solution containing KOH, iodine or an iodide, and an oxyacid, followed by contacting with a solution containing a tertiary amine.

FR 2 553 562 A1 relates to a generator of a radioactive or toxic product, for example radioactive iodine, usable especially for verifying the iodine traps of nuclear power stations. In this generator the radioactive or toxic product is formed by reaction between a radioactive or toxic precursor compound, for example sodium iodide, and a reactant, for example methyl sulphate or molecular iodine.

US 2006/177944 A1 describes an RI-labeled compound synthesizing system that comprises an RI material synthesizing section that synthesizes a labeled precursor by using a radioisotope; a plurality of RI compound manufacturing sections into which the labeled precursor and a reagent are introduced and in which a radioisotope-labeled compound is manufactured; and a switching device that selects an RI compound manufacturing section from among the plurality of RI compound manufacturing sections into which the labeled precursor is introduced.

## SUMMARY OF THE INVENTION

Aiming at the above-mentioned drawbacks in the prior art, the objective of this invention is to provide a reagent for testing the purification capacity of radioactive gas in nuclear power plant. Wherein, such reagent is generated in a field reaction in accordance with the usage. Besides, it causes no harm or little harm to the nuclear grade impregnated activated carbon of an iodide filter.

Aiming at the above-mentioned drawbacks in the prior art, a further objective of this invention is to provide a method for preparing the reagent for testing the purification capacity of radioactive gas in nuclear power plant. In this method, the reactants are non-toxic, the reaction can take place quickly and efficiently without high temperature and high pressure, and the products except methyl iodide cause no harm or little harm to the nuclear grade impregnated activated carbon of an iodide filter.

Another objective of this invention is to provide an iodide filter testing equipment of nuclear power plant which uses such reagent. Herein, the iodide filter testing equipment has good safety performance, enhanced reaction controllability and lossless iodine source.

The objective of this invention is yet to provide an iodide filter testing equipment of nuclear power plant which uses such reagent. Herein, the iodide filter testing equipment can carry out an automatic processing when a blockage failure takes place below the air entry of an injection pump in the reaction loop.

The technical solution for solving the technical problem in this invention is as follows:

> A method for preparing the reagent for testing the purification capacity of radioactive gas in nuclear power plant is provided, which comprises taking a methyl phosphate compound or a dimethyl acetal compound, an acetonitrile, a trimethyl chlorosilane and a radioactive iodine source as raw materials, mixing the raw materials and carrying out a reaction at 20~50°C for 10~40min to obtain the radioactive methyl iodide tracer for field test of iodide filter.

The volume ratio of the methyl phosphate compound or the dimethyl acetal compound, the acetonitrile and the trimethyl chlorosilane is 0.1~2: 3~6: 0.1~2.

Based on the different selection of the radioactive iodine source, the following two specific technical solutions are adopted in the preparation method of this invention:

> In a first kind of specific technical solution, the radioactive iodine source is a radioactive iodide which has a radioactivity of 10~400MBq.

In the first kind of specific technical solution, the radioactive iodide is radioactive potassium iodide or radioactive sodium iodide.

In the first kind of specific technical solution, the radioactive iodide, the acetonitrile, the methyl phosphate compound and the trimethyl chlorosilane are mixed together as the raw materials. After that, the raw materials react with each other under the blow of inert gas or under stirring; wherein a reaction temperature is 20-30°C and a reaction time is 10~30min.

In the first kind of specific technical solution, preferably: the acetonitrile and the radioactive potassium iodide are firstly added into a reaction flask in sequence, a ethyl dimethylphosphonoacetate and the trimethyl chlorosilane are then added into the reaction flask and further mixed together, and then the reagent is obtained after a reaction under stirring; wherein the reaction temperature is 30°C, the reaction time is 15 min, and the ratio between the acetonitrile, the radioactive potassium iodide, the ethyl dimethylphosphonoacetate and the trimethyl chlorosilane is 3~6ml :3~4g :0.1~2ml :0.1~2ml.

In the first kind of specific technical solution, further preferably: the acetonitrile and the radioactive potassium iodide are firstly added into a reaction flask in sequence, an O-chlorophenyl dimethyl phosphate and the trimethyl chlorosilane are then added into the reaction flask and further mixed together, and then the reagent is obtained after a reaction under stirring; wherein the reaction temperature is 25 °C, the reaction time is 10 min, and the ratio between the acetonitrile, the radioactive potassium iodide, the O-chlorophenyl dimethyl phosphate and the trimethyl chlorosilane is 3~6ml :3~4g :0.1~2ml :0.1~2ml

In the first kind of specific technical solution, the radioactive iodide, the acetonitrile, the dimethyl acetal compound and the trimethyl chlorosilane are mixed together as the raw materials. After that, the raw materials react with each other under the blow of inert gas or under stirring; wherein the reaction temperature is 40-50 °C and the reaction time is 20~40min

In the first kind of specific technical solution, preferably: the acetonitrile and the radioactive potassium iodide are firstly added into a reaction flask in sequence, a benzaldehyde dimethyl acetal and the trimethyl chlorosilane are then added into the reaction flask and further mixed together, and then the reagent is obtained after a reaction under stirring; wherein the reaction temperature is 40 °C, the reaction time is 20 min, and the ratio between the acetonitrile, the radioactive potassium iodide, the benzaldehyde dimethyl acetal and the trimethyl chlorosilane is 3~6ml : 3~4g : 0.1~2ml : 0.1~2ml.

In the first kind of specific technical solution, further preferably: the acetonitrile and the radioactive potassium iodide are firstly added into a reaction flask in sequence, a $(CH_2)_5C(OCH_3)_2$ and the trimethyl chlorosilane are then added into the reaction flask and further mixed together, and then the reagent is obtained after a reaction under stirring; wherein the reaction temperature is 45°C, the reaction time is 30 min, and the ratio between the acetonitrile, the radioactive potassium iodide, the $(CH_2)_5C(OCH_3)_2$ and the trimethyl chlorosilane is 3~6ml : 3~4g : 0.1~2ml : 0.1~2ml.

In a second kind of specific technical solution, the radioactive iodine source is prepared by mixing a buffer distribution solution with the water solution of radioactive iodide. The radioactivity of the prepared radioactive iodine source is 10~400MBq. The buffer distribution solution is prepared by mixing a non-radioactive iodide with an acetone.

In the second kind of specific technical solution, the radioactive iodide is radioactive potassium iodide or radioactive sodium iodide, and the non-radioactive iodide is non-radioactive potassium iodide or non-radioactive sodium iodide.

In the second kind of specific technical solution, it comprises the following steps:

Step (1), mixing the acetonitrile and the acetone, and then adding the non-radioactive iodide to obtain a mixed solution of the acetonitrile and the buffer distribution solution, wherein the ration between the acetonitrile, the non-radioactive iodide and the buffer distribution solution is 1~4ml : 0.1~5g : 0.5~2ml;

Step (2), adding the water solution of radioactive iodide into the mixed solution of step (1) to obtain a mixture of the acetonitrile and the radioactive iodine source;

Step (3), adding the methyl phosphate compound or the dimethyl acetal compound and the trimethyl chlorosilane into the mixture of the acetonitrile and the radioactive iodine source of step (2), mixing such raw materials and carrying out the reaction to obtain the radioactive methyl iodide tracer for field test of iodide filter after the reaction under the blow of inert gas or under stirring; wherein the reaction temperature is 20-50 °C and the reaction time is 10 ~40min.

In the second kind of specific technical solution, in step (3), the methyl phosphate compound and the trimethyl chlorosilane are added into the mixture of the radioactive iodine source and the acetonitrile and further mixed together. After that, the raw materials react with each other under the blow of inert gas or under stirring; wherein the reaction temperature is 20-30°C and the reaction time is 10 ~30min.

In the second kind of specific technical solution, in step (3), the dimethyl acetal compound and the trimethyl chlorosilane are added into the mixture of the radioactive iodine source and the acetonitrile and further mixed together. After that, the raw materials react with each other under the blow of inert gas or under stirring; wherein the reaction temperature is 40-50°C and the reaction time is 20~40min.

In all of the above-mentioned methods for preparing the reagent for testing the purification capacity of radioactive

gas in nuclear power plant, the methyl phosphate compound is $R_1PO(OCH_3)_2$, wherein $R_1$ is $PhClCH_2$, $CCl_3$, $NCCH_2$, MeCO, $MeOCOCH_2$, $EtOCOCH_2$, t-$BuOCOCH_2$, $PhCH_2OCOCH_2$, EtOCO, $Et_2NCO$, MeCOOCHPh, $PhCOCH_2$, $MeOCH_2$, $(MeO)_2CHCH_2$ or $(MeO)_2CH$.

In all of the above-mentioned methods for preparing the reagent for testing the purification capacity of radioactive gas in nuclear power plant, the dimethyl acetal compound is $R_2R_3C(OCH_3)_2$, wherein $R_2$ is Ph, $Me(CH_2)_5$ or $(CH_2)_5$, and $R_3$ is Me or H.

An iodide filter testing equipment of nuclear power plant which is specially adapted to use a reagent which is prepared by taking a methyl phosphate compound or a dimethyl acetal compound, an acetonitrile, a trimethyl chlorosilane and a radioactive iodine source as raw materials, mixing said raw materials and carrying out a reaction at 20~50°C; the iodide filter testing equipment comprises a negative pressure box, a compressed air distributor located on the negative pressure box, a methyl iodide generator and a pneumatic control unit located inside the negative pressure box. The methyl iodide generator comprises a compressed air injection pump, a check valve, a throttle, a generator body, a sleeve needle, a reaction flask, a lifting rack for reaction flask and a cylinder for moving the lifting rack up and down. The methyl iodide generator is connected with an intake pipe which is connected to the atmosphere outside the negative pressure box. The intake pipe is connected to an internal needle of the sleeve needle through the generator body, and the air entry of the compressed air injection pump is connected to the space between the internal needle and an external needle of the sleeve needle. The pneumatic control unit comprises a micro-pressure signal valve, a push-pull reversing valve, a two-position five-way one-way air control reversing valve, a one-way throttle valve and a check valve. Its characteristics lie in that, the push-pull reversing valve is a pneumatically reset one, and the intake pipe is provided with a non-negative pressure switch with time delay output and a check valve.

In the iodide filter testing equipment of nuclear power plant, the non-negative pressure switch with time delay output connects with the pneumatically reset push-pull reversing valve, wherein the time delay output non-negative pressure switch outputs gas to the pneumatically reset push-pull reversing valve to close the pneumatically reset push-pull reversing valve.

In the iodide filter testing equipment of nuclear power plant, the pneumatically reset push-pull reversing valve comprises a push-pull reversing valve cylinder. Three pistons are arranged in series inside the push-pull reversing valve cylinder, and a handle connected with the pistons for pulling or pushing the pistons is arranged outside the push-pull reversing valve cylinder. Five pores including a first pore to a fifth pore are located in the wall of the push-pull reversing valve cylinder. Both ends of the push-pull reversing valve cylinder is respectively provided with a first pressure balance hole and a second pressure balance hole used to balance the internal and external pressures of the push-pull reversing valve cylinder during the movement of the pistons. When the pneumatically reset push-pull reversing valve is in an open state by pulling out the handle, a second pore is connected to a fourth pore, the first pore is connected to a third pore, and the fifth pore is sealed by the third piston; when the pneumatically reset push-pull reversing valve is in a closed state by pushing-in the handle, the first pore is connected to the fourth pore, the second pore is connected to the fifth pore, and the third pore is sealed by the first piston.

In the iodide filter testing equipment of nuclear power plant, the piston is connected through a pull rod, and the first pressure balance hole is connected to the non-negative pressure switch with time delay output.

In the iodide filter testing equipment of nuclear power plant, the time delay output non-negative pressure switch comprises a non-negative pressure switch, wherein a time delay switch is further arranged at an outlet of the non-negative pressure switch for delaying the output of a non-negative pressure signal.

In the iodide filter testing equipment of nuclear power plant, the non-negative pressure switch comprises a cylinder block which is arranged with three pistons inside. Herein, such three pistons are arranged in interval and further formed into an integrated piston group, which is provided with springs having the same elastic force on its both sides. The cylinder block is arranged with a first air vent, a second air vent and a third air vent. Its one end is provided with a pressure conduction hole which is operable to output gas for moving the piston group. The pressure conduction hole is connected with an internal needle air intake on the generator body through a pipeline. When the pressure conduction hole is not provided with a gas input or provided with a positive pressure input, the second air vent is connected to the third air vent, and the non-negative pressure switch is in an open state; when the pressure conduction hole is provided with a negative pressure input, the first through hole is connected to the second through hole because of the movement of the piston group, and the non-negative pressure switch is in a closed state.

In the iodide filter testing equipment of nuclear power plant, the sleeve needle comprises an internal needle and an external needle which are concentric with each other, wherein said internal needle is surrounded by said external needle. The space between the internal needle and the external needle is 0.5~1.5mm.

In the iodide filter testing equipment of nuclear power plant, the internal diameter and the external diameter of the internal needle are 1~1.5mm and 1.5~2mm, respectively.

In the iodide filter testing equipment of nuclear power plant, the internal diameter and the external diameter of the external needle are 2.5~3mm and 3~3.5mm, respectively.

In the iodide filter testing equipment of nuclear power plant, an outlet at the lower end of the external needle

is slightly lower than the lower end surface of a cap of the reaction flask.

In the iodide filter testing equipment of nuclear power plant, the lower end surfaces of the internal needle and the external needle are both inclined planes.

In the iodide filter testing equipment of nuclear power plant, a storage support for storing a reagent bottle is provided inside the negative pressure box, wherein the storage support is located on a bottom plate of the negative pressure box next to the methyl iodide generator.

In the iodide filter testing equipment of nuclear power plant, the storage support made of elastic material is a flat plate rack holder with a circular hole.

In this invention, the methyl phosphate compound or the dimethyl acetal compound, the radioactive iodide, the trimethyl chlorosilane and the acetonitrile are taken as the raw materials. The reagent is obtained after mixing the raw materials and carrying out a reaction between these raw materials. The reaction principle is as follows:

$$R_1PO(OCH_3)_2 + 2Me_3SiCl + 2NaI \xrightarrow{CH_3CN} R_1PO(OSiMe_3)_2 + 2CH_3I + 2NaCl$$

$$R_2R_3C(OCH_3)_2 + Me_3SiCl + NaI \xrightarrow{CH_3CN} R_2R_3CO + CH_3I + Me_3SiOCH_3 + NaCl$$

The above-mentioned raw materials are non-toxic chemicals, the reaction temperature is relatively low to be 20-50°C specifically, and the whole reaction time is short to be 10~40min under an atmospheric pressure. In this case, it can meet the requirements on field operation. Meanwhile, any other reaction products have no influence on the nuclear grade impregnated activated carbon of the iodide filter. In addition, the reaction yield is higher, and it can reach 40~90% in general. Accordingly, through the laboratory tests and field tests, it is proved that this invention can produce qualified radioactive methyl iodide by replacing the dimethyl sulfate.

Compared with another kind of raw material dimethyl acetal compound, the reaction temperature is lower and the reaction time is shorter to be more suited for field operation when the methyl phosphate compound is used as the raw material. On the basis of the original equipment, this invention has changed the push-pull reversing valve to the pneumatically reset push-pull reversing valve, and the time delay output non-negative pressure switch and the check valve are added on the intake pipe for connecting the methyl iodide generator to the atmosphere outside of the negative pressure box. In this way, both gas and liquid cannot spill over from the loop; besides, when the blockage takes place below the air entry of the injection pump in the generation loop, the present testing equipment can stop generating the methyl iodide and separate the reaction flask from the sleeve needle to prevent the remaining iodine source from strength loss which is caused by pumping the iodide source into the internal needle pipeline. This invention can enhance the security of the methyl iodide generator and ensure no strength loss of the remaining iodide source.

Compared with the prior art, this invention has made the following improvements on the sleeve needle: reducing the length and increasing the diameter of the external needle. By shortening the length of the external needle, the external needle can avoid contacting with the impurities in the liquid during stirring. On the other hand, the possibility of blocking the space between the internal and external needles due to the solid content in the liquid is reduced by increasing the diameter of the external needle. The lower end surfaces of the internal needle and the external needle are design as the inclined plane, in which case on one hand the needles are easy to puncture a rubber layer of a bottle stopper, on the one hand the needles are difficult to be blocked by the impurities in the liquid or rubber crumb of the bottle stopper.

BRIEF DESCRIPTION OF THE DRAWINGS

This invention will be further described with reference to the accompanying drawings and embodiments in the following. In the Figures:

Figure 1 is a structural diagram for an embodiment of this invention;

Figure 2 is a diagram illustrating the non-negative pressure switch with time delay output in a non-negative pressure state in an embodiment of this invention;

Figure 3 is a diagram illustrating the non-negative pressure switch with time delay output in a negative pressure state in an embodiment of this invention;

Figure 4 is a diagram illustrating the pneumatically reset push-pull reversing valve in a closed state in an embodiment of this invention;

Figure 5 is a diagram illustrating the pneumatically reset push-pull reversing valve in an open state in an embodiment of this invention; and

Figure 6 is a structural diagram for the storage support in an embodiment of this invention.

DETAILED DESCRIPTION OF THE PREFERRED EMBODIMENT

For the simulated experiment in the laboratory, non-radioactive methyl iodide is employed since the radioactive methyl iodide in the reagent is harmful to human health. It is pointed out that, the conclusion when using the non-radioactive methyl iodide for experiment in embodiments 1~13 is completely suitable for that of the radioactive iodine compound when it participates in the same reaction. Besides, the yield of the radioactive methyl iodide is the same as that of the non-radioactive methyl iodide.

Embodiment 1, 8 ml acetonitrile and 5 g non-radioactive potassium iodide are sequentially added into a 50ml round-bottomed reaction flask, wherein the non-radioactive potassium iodide is used to simulate a radioactive iodine source (similarly hereinafter, which is not repeated in the following embodiments); subsequently, 2.5 ml ethyl dimethylphosphonoacetate and 3.8 ml trimethyl chlorosilane are added into the reaction flask and further mixed to be uniform; after that, such chemicals react with each other at 30°C for 15min under a magnetic stirring, and the reagent for testing purification capacity of radioactive gas in nuclear power plant is obtained thereafter. A reflux condensing is carried out on the reagent at 70°C for 1.5h to collect the non-radioactive methyl iodide. According to a density analysis, the yield of the non-radioactive methyl iodide in the collected liquid is about 46%.

Embodiment 2, 10 ml acetonitrile and 5 g non-radioactive potassium iodide are sequentially added into a 50ml round-bottomed reaction flask; subsequently, 2.75 ml O-chlorophenyl dimethyl phosphate and 3.8 ml trimethyl chlorosilane are added into the reaction flask and further mixed to be uniform; after that, such chemicals react with each other at 25 °C for 10min under a magnetic stirring, and the reagent for testing purification capacity of radioactive gas in nuclear power plant is obtained thereafter. A reflux condensing is carried out on the reagent at 70 °C for 1.5h to collect the non-radioactive methyl iodide. According to a gas chromatography analysis, the yield of the non-radioactive methyl iodide is about 55%.

Embodiment 3, 10 ml acetonitrile and 5 g non-radioactive potassium iodide are sequentially added into a 50ml round-bottomed reaction flask; subsequently, 3 ml $CCl_3PO(OCH_3)_2$ and 4 ml trimethyl chlorosilane are added into the reaction flask and further mixed to be uniform; after that, such chemicals react with each other at 25 °C for 15min under a magnetic stirring, and the reagent for testing purification capacity of radioactive gas in nuclear power plant is obtained thereafter. A reflux condensing is carried out on the reagent at 70°C for 1.5h to collect the non-radioactive methyl iodide. According to a gas chromatography analysis, the yield of the non-radioactive methyl iodide is about 51 %.

Embodiment 4, 10 ml acetonitrile and 5 g non-radioactive potassium iodide are sequentially added into a 50ml round-bottomed reaction flask; subsequently, 3 ml $t\text{-}BuOCOCH_2PO(CCH_3)_2$ and 4 ml trimethyl chlorosilane are added into the reaction flask and further mixed to be uniform; after that, such chemicals react with each other at 25°C for 15min under a magnetic stirring, and the reagent for testing purification capacity of radioactive gas in nuclear power plant is obtained thereafter. A reflux condensing is carried out on the reagent at 70°C for 1.5h to collect the non-radioactive methyl iodide. According to a gas chromatography analysis, the yield of the non-radioactive methyl iodide is about 46%.

Embodiment 5, 10 ml acetonitrile and 5 g non-radioactive potassium iodide are sequentially added into a 50ml round-bottomed reaction flask; subsequently, 3 ml $MeCOOCHPhPOC(CH_3)_2$ and 4 ml trimethyl chlorosilane are added into the reaction flask and further mixed to be uniform; after that, such chemicals react with each other at 25 °C for 15min under a magnetic stirring, and the reagent for testing purification capacity of radioactive gas in nuclear power plant is obtained thereafter. A reflux condensing is carried out on the reagent at 70°C for 1.5h to collect the non-radioactive methyl iodide. According to a gas chromatography analysis, the yield of the non-radioactive methyl iodide is about 50%.

Embodiment 6, 10 ml acetonitrile and 5 g non-radioactive potassium iodide are sequentially added into a 50ml round-bottomed reaction flask; subsequently, 3 ml $PhCOCH_2PO(OCH_3)_2$ and 4 ml trimethyl chlorosilane are added into the reaction flask and further mixed to be uniform; after that, such chemicals react with each other at 25 °C for 15min under a magnetic stirring, and the reagent for testing purification capacity of radioactive gas in nuclear power plant is obtained thereafter. A reflux condensing is carried out on the reagent at 70°C for 1.5h to collect the non-radioactive methyl iodide. According to a gas chromatography analysis, the yield of the non-radioactive methyl iodide is about 48%.

Embodiment 7, 10 ml acetonitrile and 5 g non-radioactive potassium iodide are sequentially added into a 50ml round-bottomed reaction flask; subsequently, 3 ml $(MeO)_2CHCH_2PO(OCH_3)_2$ and 4 ml trimethyl chlorosilane are added into the reaction flask and further mixed to be uniform; after that, such chemicals react with each other at 25 °C for 10min under a magnetic stirring, and the reagent for testing purification capacity of radioactive gas in nuclear power plant is obtained thereafter. A reflux condensing is carried out on the reagent at 70°C for 1.5h to collect the non-radioactive methyl iodide. According to a gas chromatography analysis, the yield of the non-radioactive methyl iodide is about 56%.

Embodiment 8, 1 ml acetonitrile, 1ml aceton and a water solution of 0.1g non-radioactive potassium iodide are sequentially added into a 25ml round-bottomed reaction flask; subsequently, 1 ml trimethyl phosphonoacetate and 0.5 ml trimethyl chlorosilane are added into the reaction flash and further mixed to be uniform; after that, such chemicals react with each other at 20 °C for 20 min, and the reagent for testing purification capacity of radioactive gas in nuclear power plant is obtained thereafter. The gaseous non-radioactive methyl iodide is collected under the blow and carriage of nitrogen. According to a gas chromatography analysis, the yield of the non-radioactive methyl iodide in the collected

gas is about 56%.

Embodiment 9, 1 ml acetonitrile, 1ml aceton and a water solution of 0.1g non-radioactive potassium iodide are sequentially added into a 25ml round-bottomed reaction flask; subsequently, 1 ml trimethyl phosphonoacetate and 0.5 ml trimethyl chlorosilane are added into the reaction flash and further mixed to be uniform; after that, such chemicals react with each other at 30°C for 30 min, and the reagent for testing purification capacity of radioactive gas in nuclear power plant is obtained thereafter. The gaseous non-radioactive methyl iodide is collected under the blow and carriage of nitrogen. According to a gas chromatography analysis, the yield of the non-radioactive methyl iodide in the collected gas is about 74%.

Embodiment 10, 8 ml acetonitrile and 5 g non-radioactive potassium iodide are sequentially added into a 50ml round-bottomed reaction flask; subsequently, 2.5 ml benzaldehyde dimethyl acetal and 3.8 ml trimethyl chlorosilane are added into the reaction flask and further mixed to be uniform; after that, such chemicals react with each other at 40°C for 20 min under a magnetic stirring, and the reagent for testing purification capacity of radioactive gas in nuclear power plant is obtained thereafter. A reflux condensing is carried out on the reagent at 50°C for 1.5h to collect the non-radioactive methyl iodide. According to a density analysis, the yield of the non-radioactive methyl iodide in the collected liquid is about 40%.

Embodiment 11, 8 ml acetonitrile and 5 g non-radioactive potassium iodide are sequentially added into a 50ml round-bottomed reaction flask; subsequently, 2.5 ml benzaldehyde dimethyl acetal and 3.8 ml trimethyl chlorosilane are added into the reaction flask and further mixed to be uniform; after that, such chemicals react with each other at 50°C for 40 min under a magnetic stirring, and the reagent for testing purification capacity of radioactive gas in nuclear power plant is obtained thereafter. A reflux condensing is carried out on the reagent at 70 °C for 1.5h to collect the non-radioactive methyl iodide. According to a density analysis, the yield of the non-radioactive methyl iodide in the collected liquid is about 78%.

Embodiment 12, 8 ml acetonitrile and 5 g non-radioactive potassium iodide are sequentially added into a 50ml round-bottomed reaction flask; subsequently, 3 ml $Me(CH_2)_2CH(OCH_3)_2$ and 4 ml trimethyl chlorosilane are added into the reaction flask and further mixed to be uniform; after that, such chemicals react with each other at 45 °C for 30 min under a magnetic stirring, and the reagent for testing purification capacity of radioactive gas in nuclear power plant is obtained thereafter. A reflux condensing is carried out on the reagent at 50°C for 1.5h to collect the non-radioactive methyl iodide. According to a density analysis, the yield of the non-radioactive methyl iodide in the collected liquid is about 56%.

Embodiment 13, 8 ml acetonitrile and 5 g non-radioactive potassium iodide are sequentially added into a 50 ml round-bottomed reaction flask; subsequently, 3 ml $(CH_2)_5C(OCH_3)_2$ and 4 ml trimethyl chlorosilane are added into the reaction flask and further mixed to be uniform; after that, such chemicals react with each other at 45 °C for 30 min under a magnetic stirring, and the reagent for testing purification capacity of radioactive gas in nuclear power plant is obtained thereafter. A reflux condensing is carried out on the reagent at 50°C for 1.5h to collect the non-radioactive methyl iodide. According to a density analysis, the yield of the non-radioactive methyl iodide in the collected liquid is about 54%.

Embodiment 14, in the field test of the iodide filter, the methods of preparation and application for the reagent (radioactive methyl iodide) according to an embodiment of this invention are as follow:

According to a first aspect, it relates to the preparation of the mixture of acetonitrile and radioactive iodine source. At present, the radioactive iodide used in the test of iodide filter in the nuclear power plant is a commercially available pharmaceutical grade water solution of radioactive sodium iodide. For this reason, reactions in this invention need to be carried on under a anhydrous neutral condition, which means the water solution of radioactive sodium iodide cannot be used as the radioactive iodine source directly. Accordingly, a mixed solution comprising the acetone and the acetonitrile is firstly obtained according to a volume ratio of 1:1~4. The specific volume ratio between the two can be any one of such range value, wherein the ration is selected to be 1:4 in this embodiment. Subsequently, 0.1g non-radioactive sodium iodide is added into 4ml mixed solution to obtain a mixture of acetonitrile and buffer distribution solution. After that, a water solution of radioactive sodium iodide is added and mixed with the mixture, and the iodine source with desired radioactivity for the test is obtained by means of volume distribution.

According to a second aspect, in the field test of the iodide filter, the methods of preparation and application for the reagent (radioactive methyl iodide) according to an embodiment of this invention are as follow:

1, Preparation of Reaction Reagent:

(1) a trimethyl chlorosilane, a trimethyl phosphonoacetate and the iodine source prepared according to the above-mentioned preparation method of iodine source are encapsulated in a 10ml glass bottle respectively, wherein all the encapsulated trimethyl chlorosilane, the trimethyl phosphonoacetate and the iodine source have

a volume of less than 10ml, respectively.

(2) 6~8ml clear water is encapsulated in a 10ml glass bottle.

2, Checking and Cleaning of Generation Loop

The methyl iodide generator is connected with a tracer injection port of a ventilation system and it is further connected with a compressed air source. After that, a negative pressure is produced inside the box of the methyl iodide generator and a clear water bottle is placed on a bracket. The generation loop is started thereafter, wherein a reversing valve is operated to lift the bracket and make a needle pass through a bottle stopper so that the bubbling on a liquid level of the reaction bottle is observed. In this case, in the event of normal bubbling, the loop will be purged for 1min; on the contrary, a checking and removing of blocking are carried out in the loop. When the generation loop is ensured to be clear, the generation loop is stopped and the clear water bottle is taken down for standby application.

3, Generation Operation of Reagent (Radioactive Methyl Iodide)

At room temperature, three bottles respectively containing the mixed solution of radioactive iodine source and acetonitrile, the trimethyl chlorosilane and the trimethyl phosphonoacetate are placed in a negative pressure box, and then about 1 ml trimethyl chlorosilane and 1 ml trimethyl phosphonoacetate are respectively pumped out and further added into the bottle containing the mixed solution of radioactive iodine source and acetonitrile. Subsequently, this bottle is placed on a bracket after shaking, which is further kept standing for 5min before starting the generation loop. When the generation loop is started, a reversing valve is operated to lift the bracket and make a needle pass through a bottle stopper. Then the liquid within the bottle bubbles and reacts to generate the radioactive methyl iodide, and the generated radioactive methyl iodide is injected into the system.

4, Generation Termination of Radioactive Methyl Iodide and Purging of Generation Loop

When the radioactive methyl iodide has been generated for 30 minutes, the reversing valve is operated to lower the bracket. Subsequently, the bottle with the mixed solution of radioactive iodine source and acetonitrile is taken down when the negative pressure box is purged for 5min. The clear water bottle is placed onto the bracket thereafter. After that, the generation loop is started, wherein the reversing valve is operated to lift the bracket and make the needle pass through the bottle stopper so that the generation loop is purged by the clear water and the bubbling on a liquid level of the reaction bottle is observed. In this case, in the event of normal bubbling, the loop will be purged for only 8min; on the contrary, a checking and removing of blocking are carried out in the loop.

Embodiment 15, Test of Application Effect of Reagent in according to an embodiment of this invention :

According to a first aspect, a field contrast test is as follows:

Contrast tests are performed on an iodide filter of an exhaust gas treatment system (TEG) of the Daya Bay and Ling-ao nuclear power plant by utilization of the following chemicals: the product prepared according to the method of "dimethyl sulfate" and the reagent generated in this invention.

Brief description of test conditions is as follows:

1. Regarding the method of dimethyl sulfate, approximately 1ml dimethyl sulfate and 4ml aqueous iodine source of radioactive sodium iodide with a radioactivity of10MBq are used every time;
2. According to an embodiment of this invention, approximately 1ml trimethyl chlorosilane, 1ml trimethyl phosphonoacetate and 4ml mixed solution of acetonitrile and radioactive iodine source with a radioactivity of10MBq are used every time;
3. The test temperature is about 25°C;
4. Test air volume: 2000$m^3$/H$\pm$10%;
5. System injection time of radioactive methyl iodide: 30 minutes;
6. Upstream sampling time: 45 minutes;
7. Downstream sampling time: 60 minutes;

The test results are shown in Table 1.

Table 1 Results of Contrast Test

| test system | test type | purification coefficient | purification efficiency | evaluation of results |
|---|---|---|---|---|
| L9TEG001PI | dimethyl sulfate | 410 | 99.756 | better consistency |
| | this invention | 403 | 99.752 | |

(continued)

| test system | test type | purification coefficient | purification efficiency | evaluation of results |
|---|---|---|---|---|
| L9TEG002PI | dimethyl sulfate | 449 | 99.777 | good consistency |
| | this invention | 398 | 99.749 | |
| D9TEG001PI | dimethyl sulfate | 1505 | 99.934 | better consistency |
| | this invention | 1565 | 99.936 | |
| D9TEG002PI | dimethyl sulfate | 5941 | 99.983 | better consistency |
| | this invention | 6648 | 99.985 | |

Note: 1, purification coefficient (CE)

$$CE = \frac{A}{a}$$

In the formula: A-radioactivity of tracer in the upstream of iodide filter;
a-radioactivity of tracer in the downstream of iodide filter
2, purification efficiency (R):

$$R = \left(1 - \frac{a}{A}\right) \times 100 \quad (\%)$$

Test Conclusion: the purification efficiencies of such two methods in the contrast test are substantially comparable with respect to the same iodide filter. Accordingly, the radioactive methyl iodide in this invention can completely replace the radioactive methyl iodide generated by the method of dimethyl sulfate. That is, it is suited for field test of iodide filter.

Second, Influence of Iodine Source Solution on Yield of Methyl Iodide:

The radioactive iodine source for the field test is the water solution of radioactive NaI or the water solution of radioactive KI. NaI or KI can also dissolve in water, ethanol and acetone. It is easy for the trimethyl chlorosilane to have a hydrolysis reaction and an alcoholysis reaction in the presence of water or alchol, so the method for preparing the radioactive methyl iodide by usage of the trimethy chlorosilane/NaI or trimethy chlorosilane/KI is carried out under an anhydrous neutral condition. To examine the effect of solvents such as water on the above-mentioned reaction, the effects of water, ethanol and acetone on the yield of methyl iodide are studied respectively, wherein the test results are shown in Table 2.

Table 2 Effect of Different Solvents on the Yield of Methyl Iodide

| test condition | amount of KI | times | temperature | yield of gaseous methyl iodide/% |
|---|---|---|---|---|
| 1, effect of 1ml distilled water on the yield of methyl iodide | 5g | 1 | 30°C | 24.2 |
| | | 2 | 25°C | 36.3 |
| | | 3 | 25°C | 33.5 |
| | | 4 | 20°C | 34.3 |
| | 0.1g | 1 | 25°C | 10.9 |
| 2, effect of 1ml absolute ethanol on the yield of methyl iodide | 5g | 1 | 25°C | 43.0 |
| | | 2 | 25°C | 17.7 |
| | | 3 | 25°C | 38.2 |
| 3, effect of 1ml acetone on the yield of methyl iodide | 0.1g | 1 | 25°C | 86.5 |
| | | 2 | 25°C | 87.1 |

(continued)

| test condition | amount of KI | times | temperature | yield of gaseous methyl iodide/% |
|---|---|---|---|---|
| 4, effect of water with different volumes on the yield of methyl iodide when taking acetone as the solvent | 0.1g | 0.5ml water | 25°C | 34.9 |
| | | 0.4ml water | | 44.2 |
| | | 0.3ml water | | 55.6 |
| | | 0.1ml water | | 72.6 |

It can be seen from the yield of methyl iodide in table 1 that: when taking the distilled water or the ethanol as the solvent, the yield of methyl iodide is below 45%; but when taking the acetone as the solvent, the yield of methyl iodide is over 80%. Therefore, the acetone is selected as the solvent of the radioactive NaI or KI.

The volume of the water solution of radioactive NaI is generally less than 1.5 ml based on the statistics of multiple purchase records. Moreover, after the dilution with the buffer and distribution solutions, the amount of water in the radioactive solution is minimal in the actual test. The test results also show that the yield of methyl iodide is still over 70% in the presence of 0.1ml water. Accordingly, the effect of the water amount of the purchased radioactive source on the yield of methyl iodide can be neglected after dilution with acetone.

Third, Influence of Chemical Reaction Reagent on Activated Carbon

In the four kinds of organic chemical reagents participated in the reaction, only the acetone and acetonitrile may affect the absorption efficiency of a nuclear grade impregnated activated carbon to the radioactive methyl iodine. Therefore, the following tests are to verify its extent of influence.

Test condition: referring to the standard test method and field test condition of ASTM D3803 nuclear grade impregnated activated carbon.
Temperature: 25~30°C
Humidity: <40%
Test carbon bed: 5cm in total depth of carbon bed, three layers and 5cm in diameter
Feeding speed of methyl iodide: 12.2 ± 0.3m/min
Flow of methyl iodide: 1.75mg/$m^3$
Amount of radioactive methyl iodide: 0.3 ~ 2MBq
Injection time: 1 h

Test (1): Radioactive Methyl Iodine Adsorption Efficiency of Nuclear Grade Impregnated Activated Carbon

When testing the influence of the acetone and the acetonitrile on the radioactive methyl iodine adsorption efficiency of the nuclear grade impregnated activated carbon, the test for the radioactive methyl iodine adsorption efficiency of a same batched nuclear grade impregnated activated carbon is firstly carried on, wherein the results are shown in the test(1) of Table 3.

Test (2): Test for the Influence of Acetonitrile on the Radioactive Methyl Iodine Absorption Efficiency of Nuclear Grade Impregnated Activated Carbon

During the test, the injection mass ration between the acetonitrile and the radioactive methyl iodide is as follows: $m_{acetonitril}e:m_{radioactive\ methyl\ iodide}$ = 2.5:1. The results are shown in the test (2) of Table 3.

Test (3): Test for the Influence of Injecting Methyl Iodide, Acetone and Acetonitrile Simultaneously

During the test, the injection mass ration between the acetone, the acetonitrile and the radioactive methyl iodide is as follows: $m_{acetone}:m_{acetonitrile}:m_{radioactive}$ methyl iodide = 10:10:1. The results are shown in the test (3) of Table 3.

Test (4): The acetone and the acetonitrile are injected firstly, and then the radioactive methyl iodide is injected. The influence of such injection condition on the absorption efficiency of nuclear grade impregnated activated carbon to

the radioactive methyl iodine is evaluated. Herein, the amount of each reagent is the same as that of test (3), and the results are shown in the test (4) of Table 3.

Table 3 Test for Influence of Acetone and Acetonitrile on Activated Carbon

| Test number | Amount of injection | Form of test bed of nuclear grade impregnated activated carbon | Layer number of test bed | Mass (g) | Net counting of radioactivity | Adsorption efficiency |
|---|---|---|---|---|---|---|
| (1) | $M_{radioactive}$ methyl iodide =2.52mg | Test bed is composed of three carbon layer in series. | 1 | 11.24 | 394250 | 95.5% |
| | | | 2 | 19.65 | 18263 | 4.4% |
| | | | 3 | 21.46 | 368 | 0 |
| | | | Total mass | 52.35 | 412881 | 100% |
| (2) | $M_{radioactive}$ methyl iodide =2.52mg $M_{ethanol}$=6.3mg | | 1 | 11.24 | 553989 | 96.6% |
| | | | 2 | 19.63 | 19185 | 3.3% |
| | | | 3 | 21.10 | 434 | 0 |
| | | | Total mass | 51.97 | 573608 | 100% |
| (3) | Mradioactive methyl iodide =2.47mg $m_{acetone} = m_{acetonitrile}$ =24.7mg | | 1 | 17.5 | 81485 | 93.8% |
| | | | 2 | 18.0 | 4676 | 5.4% |
| | | | 3 | 15.5 | 651 | 0.7% |
| | | | Total mass | 50.0 | 86812 | 100% |
| (4) | | | 1 | 20.0 | 93975 | 96.9% |
| | | | 2 | 19.0 | 2929 | 3.0% |
| | | | 3 | 13.0 | 14 | 0 |
| | | | Total mass | 52.0 | 96918 | 100% |

Note: the differences of the net counting of radioactivity in test (1)~(4) is related with the content of radioactive methyl iodide in the bottle containing methyl iodine source.

It can be seen from the test results: the radioactive methyl iodine adsorption efficiency of impregnated activated carbon in every layer is substantially comparable in different conditions in test (1) ~ (4). Beside, the total adsorption efficiency of the test bed is 100%, which indicates that the acetone and the acetonitrile have no influence on the absorption efficiency of activated carbon to the radioactive methyl iodine.

Embodiment 16: as shown Figure 1, an iodide filter testing equipment of nuclear power plant comprises a negative pressure box 2. A compressed air distributor 4 is located on the negative pressure box 2 and a compressed air injection pump 21 is also connected with the negative pressure box 2. The compressed air injection pump 21 is connected with a gas supply port of the compressed air distributor 4 for maintaining a negative pressure within the negative pressure box 2. An air suction pipe of the compressed air injection pump 21 is stretched into the negative pressure box 2, wherein an air entry of the air suction pipe is equiped with an iodine remover 20.

The negative pressure box 2 is provided with a methyl iodide generator inside, which comprises a compressed air injection pump 3, a check valve 5, a throttle 6, a generator body 23, a sleeve needle 18, a reaction flask 17, a cylinder 13 and a lifting rack for reaction flask 16, wherein the lifting rack for reaction flask 16 comprises a reaction flask support for supporting the reaction flask 17 and a fixture for clamping and fixing the reaction flask 17. The compressed air distributor 4 is connected with the compressed air injection pump 3 to supply air for the latter. An air entry of the compressed air injection pump 3 is connected with the check valve 5, a lower end of the check valve 5 is connected with the throttle 6, a lower end of the throttle 6 is connected with the generator body 23, and the sleeve needle 18 is located below the generator body 23 and is fixed to the generator body 23. The cylinder 13 is installed on a bearing 14,

while its piston rod is connected with the lifting rack for reaction flask 16 to move the reaction flask 17. The generator body 23 is connected with an intake pipe 204, which comprises a rotameter 22, a check valve 27, a non-negative pressure switch 26 with time delay function, and an air intake iodine remover 1 located at the end of the intake pipe 204 outside the negative pressure box 2. The sleeve needle 18 comprises an external needle and an internal needle, wherein the air outside the negative pressure box 2 is connected to the internal needle of the sleeve needle 18 through the intake iodine remover 1 and the generator body 23, and the air entry of the compressed air injection pump 3 is connected to the space between the external needle and the internal needle of the sleeve needle 18.

As shown in Figure 1, the negative pressure box 2 is also provided with a pneumatic control unit inside, which comprises a micro-pressure signal valve 8, a pneumatically reset push-pull reversing valve 10, a one-way throttle valve 7, a check valve 9 and a two-position five-way one-way air control reversing valve 11. An air intake P of the two-position five-way one-way air control reversing valve11 is connected with a gas supply port of compressed air distributor 4, wherein such gas supply port is intended for the pneumatic control unit. A second pore A of the two-position five-way one-way air control reversing valve11 is connected with an upper air intake of the cylinder 13 through the check valve 9, its first pore B is connected with the air intake P of the pneumatically reset push-pull reversing valve 10, and its control pore K is connected with a gas outlet of the micro-pressure signal valve 8.

As shown in Figures 2-5, the non-negative pressure switch 26 comprises a cylinder block 260 which is arranged with three pistons 262, 263, 264 inside. Herein, such three pistons in interval are in a serial connection to form into an integrated piston group, which is provided with springs 216, 266 having the same elastic force on its both sides. The cylinder block 260 is arranged with a first air vent Z, a second air vent T, a third air vent E and a fourth air vent Q. The first air vent Z and the fourth air vent Q are connected to the air inside the negative pressure box 2. The third air vent E is connected with the compressed air distributor 4 so as to provide power to the non-negative pressure switch 26. One end of the cylinder block 260 is equipped with a pressure conduction hole W which is operable to input gas for moving the piston. The pressure conduction hole W is connected with the check valve 27 of the intake pipe 24 through a pipeline. As shown in Figure 2, when a non-negative pressure signal is inputted into the pressure conduction hole W, the second air vent T is connected to the third air vent E, and the compressed air is delivered to a port P1 of the pneumatically reset push-pull reversing valve 10 through the third air vent E and the second air vent T to become a power source for resetting or prevention. In this way, the handle is closed or prevented from being opened. That is, if the handle is opened originally, it will be closed; if the handle is closed originally, it will be prevented from being opened. In this case, the non-negative pressure switch is in an open state. As shown in Figure 3, when a negative pressure is inputted into the pressure conduction hole W, an air chamber with the spring 261 is under a negative pressure, resulting in that the pistons 262, 263, 264 move toward the pressure conduction hole W and the non-negative pressure switch is in a closed state. Due to the movement of the piston 262, 263, 264, the first air vent Z is connected to the second air vent T, and the air inside the pneumatically reset push-pull reversing valve 10 is discharged from a first pressure balance hole P1 through the first air vent Z of the non-negative pressure switch 26. When the compressed air enters the space between the pistons 263 and 264, the two remain still due to the same stress. Correspondingly, the compressed air does not provide power to the pneumatically reset push-pull reversing valve 10 and the non-negative pressure switch is in a closed state at this time. The fourth air vent Q is used to keep a stable pressure for the air chamber with the spring 266 during the movement of the pistons 262, 263, 264, while it is also used to reduce the air resistance caused by the movement of the pistons 262, 263, 264.

As shown in Figure 4 and Figure 5, the pneumatically reset push-pull reversing valve 10 comprises a push-pull reversing valve cylinder 101, inside which is the three pistons 102, 103, 104 are connected in series by a pull rod 106. A handle 105 for pulling or pushing the pistons 102, 103, 104 is arranged outside the push-pull reversing valve cylinder 101 and connected with the piston s 102, 103, 104. Five pores (i.e. a first pore b, a second pore a, a third pore c, a fourth pore P' and a fifth pore d) are respectively located in the wall of the push-pull reversing valve cylinder 101. Both ends of the push-pull reversing valve cylinder 101 is respectively provided with a first pressure balance hole P1 and a second pressure balance hole e used to balance the internal and external pressures of the push-pull reversing valve cylinder 101 during the movement of the pistons 102, 103, 104. The third pore c and the fifth pore are directly connected to the air inside the negative pressure box. As shown in Figure 4, when the pneumatically reset push-pull reversing valve is in a closed state by pushing-in the handle 105, the first pore b is connected to the fourth pore P', the second pore a is connected to the fifth pore d, and the third pore c is sealed by the first piston 102. As shown in Figure 5, when the pneumatically reset push-pull reversing valve 10 is in an open state by pulling out the handle 105, the second pore a is connected to the fourth pore P', the first pore b is connected to the third pore c, and the fifth pore d is sealed by the third piston 104. When the pull rod 106 is pulled or pushed through the handle 105, the gas within the air chamber between an outer side of the piston 104 and the push-pull reversing valve cylinder 101 is discharged or inhaled through e. The removal of air resistance facilitates the push and pull of the handle, thereby changing the state of the switch.

As shown in Figures 1-5, the second pore a of the pneumatically reset push-pull reversing valve 10 is connected with a lower air intake of the cylinder 13, the first pore b is connected with the upper air intake of the cylinder 13 through the one-way throttle valve 7, and the first pressure balance hole $P_1$ is connected with the second air vent T of the non-negative pressure switch. For the micro-pressure signal valve 8, its air intake M is connected with an air supply pipeline of the pneumatic control unit of the compressed air distributor 4, its signal input port N is connected with the air entry of the compressed air injection pump 3. The bottom of negative pressure box 2 is provided with a base 19 and its side is provided with a vacuum meter 25.

The operation process is as follows: the handle 105 outside the negative pressure box 2 is pulled out, wherein an upper cylinder of the cylinder 13 exhausts through the first pore b and the third pore c of the pneumatically reset push-pull reversing valve 10, and the second pore a of the pneumatic reset push-pull reversing valve 10 is connected to the fourth pore P' so as to provide air to a bottom cylinder of the cylinder 13. The reaction flask 17 is lifted to an appropriate height, so that the sleeve needle 18 equipped in the generator body 23 penetrate into a rubber plug of the reaction flask 17. In detail, the longer internal needle dips into the solution in certain depth, while the shorter external needle is above the liquid level so as to connect the gas within the reaction flask 17 to the air entry of the compressed air injection pump 3. A negative pressure is produced at the air entry of the compressed air injection pump 3 after it connects to the working compressed air. Under the action of the negative pressure, the check valve 5 is opened and a vacuum is produced inside the gas space of the reaction flask 17. Under the action of differential pressure, the air outside the negative pressure box 2 enters the internal needle through the iodine remover 1 and the intake pipeline, and then it begins to bubble in the solution of the reaction flask 17 after overcoming a liquid seal. Herein, the bubbling air is not only used as a carrier gas for methyl iodide, but also used to stir the mother solution to accelerate the reaction rate. The airflow loaded with the methyl iodide flows into the compressed air injection pump 3 through the open check valve 5 when it is stable by the throttle 6, and then it mixes with the working air and further flows out thereafter. The bubbling flow is indicated by the rotameter 22 and can be controlled by adjusting the work pressure of the compressed air injection pump 3. The carrier air inlet is provided with the iodine remover 1, aiming to prevent the residual radioactivity gas in the pipeline from escaping when the generator is not working, and to prevent the radioactive material in the reaction flask 17 from being pressed out from the air inlet to cause pollution to testers when the blockage takes place in the generation loop. In order to connect the outlet pipe of the methyl iodide with the injection port of the test loop easily, the position of the methyl iodide generator can be adjusted along a sliding rail 12 randomly. When the handle of the pneumatically reset push-pull reversing valve 10 is push inward, the upper cylinder of the cylinder 13 exhausts through the second pore a and the fifth pore d of the pneumatically reset push-pull reversing valve 10, the fourth pore P' of the pneumatically reset push-pull reversing valve 10 is connected to the first pore b so as to provide the air to the upper cylinder of the cylinder 13. The reaction flask 17 is lowered down to be separated from the sleeve needle 18, thus stopping the output of methyl iodine.

In normal situation, since a negative pressure exists at the air entry of the compressed air injection pump 3, the micro-pressure signal valve 8 does not output a command, and a port B of the two-position five-way one-way air control reversing valve11 operates to supply the gas to the port P' of the pneumatically reset push-pull reversing valve 10. Herein, in order to control the lifting of the piston of the cylinder 13 and thus to control the stop and start of the generation of gaseous methyl iodide, an operator can operate the handle of the pneumatically reset push-pull reversing valve 10 to pull it out or push it in, wherein the handle is arranged outside the negative pressure box 2. However, in the unusual cases, for example, the valve of a methyl iodide injection pipeline is not opened when outputting the methyl iodide, or the resistance of sample-injection pipeline is too high to cause a positive pressure in the air entry of the compressed air injection pump 3 due to the blockage at its air outlet. In order to prevent the radioactive gas in the reaction flask 17 from being pressed out reversely by the positive pressure, the air entry of the compressed air injection pump 3 is provided with the check valve 5; moreover, at the moment that the air entry of the compressed air injection pump 3 becomes the positive pressure, the micro-pressure signal valve 8 of the pneumatic control unit immediately works after receiving the positive pressure signal. Specifically, the micro-pressure signal valve 8 outputs a pressure to the control pore K of the two-position five-way one-way air control reversing valve11 so as to close the vent pore and cut off the air source of the pneumatically reset push-pull reversing valve 10 and the bottom cylinder of the cylinder 13. In this case, the bottom cylinder of cylinder 13 exhausts through the port B of the two-position five-way one-way air control reversing valve 11. At the same time, the gas outlet A of the two-position five-way one-way air control reversing valve11 is opened, the upper air intake of the cylinder 13 is inflated through the check valve 9, and the reaction flask 17 is lowered down to be separated from the sleeve needle 18. After that, the output of methyl iodine is stopped, which indicates the protective effect of the pneumatic control unit. The start time for the pneumatic control unit is about 1~2s. However, since the gas channel of the methyl iodide generator is provide with the check valve 5, only a small amount of air enters the reaction flask 17 reversely during this time, which basically causes no escaping of the radioactive liquid. When the abnormal cases are gone, the protection state will be released automatically, in which case the normal output of methyl iodide is restored. The check valve 27 can prevent the radioactive gas and liquid in the reaction flask 17 from flowing to the external environment reversely.

As shown in Figures 2-5, the specific action process of the non-negative pressure switch with time delay output 26 and the pneumatically reset push-pull reversing valve 10 is as follows: after the methyl iodide generator is connected to the compressed air source, the compressed air prevents the handle from being pulled out when it passes through the

compressed air distributor 4 and the third air vent E of the non-negative pressure switch with time delay output 26, and then enters the first pressure balance hole P1 of the pneumatically reset push-pull reversing valve 10 through the second air vent T. When a negative pressure is produced inside the reaction bottle 17 by the compressed air injection pump 3, the internal needle of the sleeve needle 18 pumps air from outside through the pipeline 204, the piston of the non-negative pressure switch with time delay output 26 moves to the right, the first air vent Z is connected to the second air vent T, and the first pressure balance hole P1 of the pneumatically reset push-pull reversing valve 10 exhausts through such connection. Herein, the pneumatically reset push-pull reversing valve 10 is manually pulled out, and the reaction flask support 15 is operated to move upward so as to generate and inject the methyl iodide. The two-position five-way one-way air control reversing valve 11 has no change and the pneumatically reset push-pull reversing valve 10 only increases with the pneumatic reset. Based on these reasons, if a positive pressure exists at the air entry of the compressed air injection pump 3 in the output loop of the methyl iodide, the reaction flask support 15 will be lowered down as well. Thus, the purpose to equip the non-negative pressure switch 26 with the time delay output lies in that: in the absence of time delay output, when a positive pressure exists at the compressed air injection pump 3, the reaction flask support 15 is not lowered down in time, and a non-negative pressure also occurs at the pressure conduction hole K of the non-negative pressure switch 26 immediately, thereby resetting the handle right away; in this way, when a negative pressure restores at the air entry of the compressed air injection pump 3, the reaction flask support 15 cannot be lifted automatically. In the presence of time delay output, the micro-pressure signal valve 8 is ensured to take an action at first to lower down the reaction flask support 15. After that, the non-negative pressure switch 26 is in a state of negative pressure in normal, so that it does not supply the gas to the first pressure balance hole P1 of the pneumatically reset push-pull reversing valve 10, and the handle will not be rest either. When a negative pressure restores at the air entry of the compressed air injection pump 3, the reaction bottle support 15 can rise automatically so as to carry on the injection of the methyl iodide.

When the methyl iodide generator is put into operation, the handle of the pneumatically reset push-pull reversing valve 10 has been pulled out. Accordingly, if the blockage occurs below the air entry of the injection pump 3, a non-negative pressure exists inside the reaction flask 17, the pistons 262, 263, 264 of the non-negative pressure switch 26 with time delay output move to the left, the compressed air enters the first pressure balance hole P1 of the pneumatically reset push-pull reversing valve 10 to drive the pistons 102, 103, 104 to move towards the left by passing through the distributor 4, the port E of the non-negative pressure switch 26 and the second air vent T, the first pore b is connected to the air source so that the upper cylinder of the cylinder 13 inhales the gas and the bottom cylinder exhausts through the second pore a, and further the reaction flask support 15 moves downwards so as to drive the reaction flask 17 to be separated from the sleeve needle 18. After the separation, if the rotameter has a flow indication, it indicates that the blocking position is between the sleeve needle 18 and the check valve 5. At this time, the non-negative pressure switch 26 is in a state of negative pressure, the air source is not connected to the second air vent T, the first pressure balance hole P1 of the pneumatically reset push-pull reversing valve 10 is not ventilated, and the handle can be pulled out so as to lift the reaction flask support 15. However, the pneumatic reset is carried out due to the above-mentioned protection, which can lower down the reaction flask support 15 and stop the generation process. If the rotameter 22 has no flow indication, it is indicated that the blocking position is at the internal needle of the sleeve needle 18. At this time, the non-negative pressure switch 26 is in a state of non-negative pressure, the compressed air enters by passing through the second air vent T and the first pressure balance hole P1 of the pneumatically reset push-pull reversing valve 10, and the handle cannot be pulled out. Only the above-mentioned defect is treated well, can the generation be started again.

The sleeve needle comprises the internal needle and the external needle which are shielded with each other. The space between the internal needle and the external needle is preferable to be 0.5~1.5mm, and an outlet at the lower end of the external needle is slightly lower than the lower end surface of a cap of the reaction flask. Compared with the existing sleeve needle, the length of the external needle is reduced by more than 2mm, and the diameter of the external needle is increased by 0.5~1mm. The lower end surfaces of the internal needle and the external needle are both inclined planes, which form a long and pointed triangle-shaped needle. By shortening the length of the external needle, the external needle can avoid contacting with the liquid during stirring so as to reduce the possibility of blocking the space between the internal and external needles due to the solid content in the liquid. It is easy for the needle with inclined plane to puncture a rubber layer of a bottle stopper; instead, it is difficult for the needle to be blocked by the rubber crumb of the bottle stopper..

As shown in Figure 6, a storage support 50 made of elastic material is arranged on the bottom inside the negative pressure box. The storage box has a length of 15cm, a width of 4-5cm and a thickness of 4cm. Besides, the storage support 50 is provided with four circular holes 51, wherein the diameter of two is φ 32, and the diameter of the other two are φ26. The above-mentioned circular holes are used to place reagent bottle used in tests, wherein the elastic material is easy to plug the syringe used during the preparation. The holes in the four corners of the base are in a threaded connection with the bottom plate of the negative pressure box 2 of the portable methyl iodide generator.

## Claims

1. A method for preparing a reagent for testing the purification capacity of radioactive gas in a nuclear power plant, **characterized in** taking a methyl phosphate compound or a dimethyl acetal compound, an acetonitrile, a trimethyl chlorosilane and a radioactive iodine source as raw materials; mixing said raw materials, and carrying out a reaction at a reaction temperature of 20~50°C for a reaction time of 10~40min to obtain the reagent for testing the purification capacity of radioactive gas in nuclear power plant.

2. The method for preparing the reagent for testing the purification capacity of radioactive gas in a nuclear power plant of claim 1, **characterized in that** the volume ratio of said methyl phosphate compound or said dimethyl acetal compound, said acetonitrile and said trimethyl chlorosilane is 0.1~2:3~6:0.1~2.

3. The method for preparing the reagent for testing the purification capacity of radioactive gas in a nuclear power plant of claim 2, **characterized in that** said radioactive iodine source is a radioactive iodide which has a radioactivity of 10~400MBq.

4. The method for preparing the reagent for testing the purification capacity of radioactive gas in a nuclear power plant of claim 3, **characterized in that** said radioactive iodide is radioactive potassium iodide or radioactive sodium iodide.

5. The method for preparing the reagent for testing the purification capacity of radioactive gas in a nuclear power plant of claim 4, **characterized in** taking said radioactive iodide, said acetonitrile, said methyl phosphate compound and said trimethyl chlorosilane as said raw materials, mixing said raw materials and carrying out the reaction under the blow of inert gas or under stirring; wherein a reaction temperature is 20-30°C and a reaction time is 10~30min.

6. The method for preparing the reagent for testing the purification capacity of radioactive gas in a nuclear power plant of claim 4, **characterized in** taking said radioactive iodide, said acetonitrile, said dimethyl acetal compound and said trimethyl chlorosilane as said raw materials, mixing said raw materials and carrying out the reaction under the blow of inert gas or under stirring; wherein a reaction temperature is 40-50°C and a reaction time is 20~40min.

7. The method for preparing the reagent for testing the purification capacity of radioactive gas in a nuclear power plant of claim 2, **characterized in that** said radioactive iodine source is prepared by mixing a buffer distribution solution with the water solution of radioactive iodide; the radioactivity of the prepared radioactive iodine source is 10~400MBq; the buffer distribution solution is prepared by mixing a non-radioactive iodide with an acetone.

8. The method for preparing the reagent for testing the purification capacity of radioactive gas in a nuclear power plant of claim 7, **characterized in that** said radioactive iodide is radioactive potassium iodide or radioactive sodium iodide, said non-radioactive iodide is non-radioactive potassium iodide or non-radioactive sodium iodide.

9. The method for preparing the reagent for testing the purification capacity of radioactive gas in a nuclear power plant of claim 8, **characterized in** comprising the following steps:

   step (1), mixing said acetonitrile and said acetone, and then adding said non-radioactive iodide to obtain a mixed solution of said acetonitrile and said buffer distribution solution, wherein the ration between said acetonitrile, said non-radioactive iodide and said buffer distribution solution is 1-4 ml : 0.1~5 g : 0.5~2 ml;
   step (2), adding the water solution of radioactive iodide into said mixed solution of step (1) to obtain a mixture of said acetonitrile and said radioactive iodine source;
   step (3), adding said methyl phosphate compound or said dimethyl acetal compound and said trimethyl chlorosilane into said mixture of said acetonitrile and said radioactive iodine source of step (2), mixing said raw materials and carrying out the reaction to obtain said reagent for testing the purification capacity of radioactive gas in the nuclear power plant under the blow of inert gas or under stirring; wherein a reaction temperature is 20-50°C and a reaction time is 10~40min.

10. The method for preparing the reagent for testing the purification capacity of radioactive gas in a nuclear power plant of claim 9, **characterized in** adding in said step (3) said methyl phosphate compound and said trimethyl chlorosilane into said mixture of said radioactive iodine source and said acetonitrile, mixing them to be uniform and carrying out the reaction under the blow of inert gas or under stirring; wherein the reaction temperature is 20-30°C and the reaction time is 10~30min.

**11.** The method for preparing the reagent for testing the purification capacity of radioactive gas in a nuclear power plant of claim 9, **characterized in** adding in said step (3) said dimethyl acetal compound and said trimethyl chlorosilane into said mixture of said radioactive iodine source and said acetonitrile, mixing them to be uniform and carrying out the reaction under the blow of inert gas or under stirring; wherein the reaction temperature is 40-50°C and the reaction time is 20~40min.

**12.** The method for preparing the reagent for testing the purification capacity of radioactive gas in a nuclear power plant of any of claims 1~11, **characterized in that** said methyl phosphate compound is $R_1PO(OCH_3)_2$, wherein $R_1$ is $PhClCH_2$, $CCl_3$, $NCCH_2$, MeCO, $MeOCOCH_2$, $EtOCOCH_2$, t-$BuOCOCH_2$, $PhCH_2OCOCH_2$, EtOCO, $Et_2NCO$, MeCOOCHPh, $PhCOCH_2$, $MeOCH_2$, $(MeO)_2CHCH_2$ or $(MeO)_2CH$.

**13.** The method for preparing the reagent for testing the purification capacity of radioactive gas in a nuclear power plant of any of claims 1~11, **characterized in that** said dimethyl acetal compound is $R_2R_3C(OCH_3)_2$, wherein $R_2$ is Ph, $Me(CH_2)_5$ or $(CH_2)_5$, and $R_3$ is Me or H.

**14.** The method for preparing the reagent for testing the purification capacity of radioactive gas in a nuclear power plant of any of claims 1~11, **characterized in** adding said acetonitrile and said radioactive potassium iodide into a reaction flask in sequence, then adding an ethyl dimethylphosphonoacetate and said trimethyl chlorosilane into said reaction flask and carrying out the reaction under stirring to obtain said reagent; wherein the reaction temperature is 30°C, the reaction time is 15min; the ratio between said acetonitrile, said radioactive potassium iodide, said ethyl dimethylphosphonoacetate and said trimethyl chlorosilane is 3~6ml : 3~4g : 0.1~2ml : 0.1~2ml.

**15.** The method for preparing the reagent for testing the purification capacity of radioactive gas in a nuclear power plant of any of claims 1~11, **characterized in** adding said acetonitrile and said radioactive potassium iodide into a reaction flask in sequence, then adding an O-chlorophenyl dimethyl phosphate and said trimethyl chlorosilane into said reaction flask and carrying out the reaction under stirring to obtain said reagent; wherein the reaction temperature is 25°C, the reaction time is 10 min; the ratio between said acetonitrile, said radioactive potassium iodide, said O-chlorophenyl dimethyl phosphate and said trimethyl chlorosilane is 3~6ml : 3~4g : 0.1~2m1 : 0.1~2ml.

**16.** The method for preparing the reagent for testing the purification capacity of radioactive gas in a nuclear power plant of any of claims 1~11, **characterized in** adding said acetonitrile and said radioactive potassium iodide into a reaction flask in sequence, then adding a benzaldehyde dimethyl acetal and said trimethyl chlorosilane into said reaction flask and carrying out the reaction under stirring to obtain said reagent; wherein the reaction temperature is 40°C, the reaction time is 20 min; the ratio between said acetonitrile, said radioactive potassium iodide, said benzaldehyde dimethyl acetal and said trimethyl chlorosilane is 3~6ml : 3~4g : 0.1~2ml: 0.1~2ml.

**17.** The method for preparing the reagent for testing the purification capacity of radioactive gas in a nuclear power plant of any of claims 1~11, **characterized in** adding said acetonitrile and said radioactive potassium iodide into a reaction flask in sequence, then adding a $(CH_2)_5C(OCH_3)_2$ and said trimethyl chlorosilane into said reaction flask and carrying out the reaction under stirring to obtain said reagent; wherein the reaction temperature is 45°C, the reaction time is 30 min; the ratio between said acetonitrile, said radioactive potassium iodide, said $(CH_2)_5C(OCH_3)_2$ and said trimethyl chlorosilane is 3~6ml : 3~4g : 0.1~2ml : 0.1~2ml.

**18.** An iodide filter testing equipment for a nuclear power plant which is specially adapted to use a reagent which is prepared by taking a methyl phosphate compound or a dimethyl acetal compound, an acetonitrile, a trimethyl chlorosilane and a radioactive iodine source as raw materials, mixing said raw materials and carrying out a reaction at 20~50°C; the iodide filter testing equipment comprising a negative pressure box (2), a compressed air distributor (4) located on the negative pressure box (2), a methyl iodide generator and a pneumatic control unit located inside the negative pressure box (2); wherein the methyl iodide generator comprises a compressed air injection pump (3), a check valve (5), a throttle (6), a generator body (23), a sleeve needle (18), a reaction flask (17), a lifting rack for reaction flask (16) and a cylinder (13) for moving the lifting rack up and down; the methyl iodide generator is connected with an intake pipe (204) which is connected to the atmosphere outside said negative pressure box (2); said intake pipe (204) is connected to an internal needle of said sleeve needle (18) through said generator body (23), and an air entry of said compressed air injection pump (3) is connected to a space between said internal needle and an external needle of said sleeve needle (18); said pneumatic control unit comprises a micro-pressure signal valve (8), a push-pull reversing valve (10), a two-position five-way one-way air control reversing valve (11), a one-way throttle valve (7) and a check valve (9); **characterized in that**, said push-pull reversing valve (10) is a pneumatically reset one, and said intake pipe (204) is provided with a non-negative pressure switch (26) with time delay output and a

check valve (27).

**19.** The iodide filter testing equipment for a nuclear power plant of claim 18, **characterized in that** said non-negative pressure switch (26) with time delay output connects with said pneumatically reset push-pull reversing valve (10), wherein said non-negative pressure switch (26) with time delay output outputs gas to said pneumatically reset push-pull reversing valve (10) to close said pneumatically reset push-pull reversing valve (10).

**20.** The iodide filter testing equipment for a nuclear power plant of claim 19, **characterized in that** said pneumatically reset push-pull reversing valve (10) comprises a push-pull reversing valve cylinder (101); wherein three pistons (102, 103, 104) are arranged in series inside said push-pull reversing valve cylinder (101), and a handle (105) connected with said pistons (102, 103, 104) for pulling or pushing said pistons (102, 103, 104) is arranged outside said push-pull reversing valve cylinder (101); five pores (b, a, c, P', d) including a first pore (b) to a fifth pore (d) are located in the wall of said push-pull reversing valve cylinder (101); both ends of said push-pull reversing valve cylinder (101) is respectively provided with a first pressure balance hole (P1) and a second pressure balance hole (e) used to balance the internal and external pressures of said push-pull reversing valve cylinder (101) during the movement of said pistons (102, 103, 104); when said pneumatically reset push-pull reversing valve (10) is in an open state by pulling out said handle (105), a second pore (a) is connected to a fourth pore (P'), said first pore (b) is connected to a third pore (c), and said fifth pore (d) is sealed by the third piston (104); when said pneumatically reset push-pull reversing valve (10) is in a closed state by pushing-in said handle (105), said first pore (b) is connected to said fourth pore (P'), said second pore (a) is connected to said fifth pore (d), and said third pore (c) is sealed by said first piston (102); said piston is connected through a pull rod (106), and said first pressure balance hole (P1) is connected to said non-negative pressure switch (26) with time delay output.

**21.** The testing equipment of iodide filter for a nuclear power plant according to any of claims 18-20, **characterized in that** said non-negative pressure switch (26) with time delay output comprises a non-negative pressure switch (26), wherein a time delay switch is further arranged at an outlet of said non-negative pressure switch (26) for delaying the output of a non-negative pressure signal;
said non-negative pressure switch (26) comprises a cylinder block (260) which is arranged with three pistons inside (262, 263, 264); said three pistons (262, 263, 264) are arranged in interval and further formed into an integrated piston group, which is provided with springs (216, 266) having the same elastic force on its both sides; said cylinder block (260) is arranged with a first air vent (Z), a second air vent (T) and a third air vent (E); one end of said cylinder block (260) is provided with a pressure conduction hole (W) which is operable to output gas for moving the piston group; said pressure conduction hole (W) is connected with an internal needle air intake on said generator body (23) through a pipeline; when said pressure conduction hole (W) is not provided with a gas input or provided with a positive pressure input, said second air vent (T) is connected to said third air vent (E), and said non-negative pressure switch (26) is in an open state; when said pressure conduction hole (W) is provided with a negative pressure input, said first through hole is connected to said second through hole because of the movement of said piston group, and said non-negative pressure switch (26) is in a closed state.

**22.** The iodide filter testing equipment for a nuclear power plant of any of claims 18-20, **characterized in that** said sleeve needle (18) comprises an internal needle and an external needle which are concentric with each other, wherein said internal needle is surrounded by said external needle; said space between said internal needle and said external needle is 0.5~1.5mm.

**Patentansprüche**

**1.** Verfahren zum Herstellen eines Reagens zum Testen des Reinigungsvermögens von radioaktivem Gas in einem Kernkraftwerk,

**gekennzeichnet durch**
Verwenden einer Methylphosphatverbindung oder einer Dimethylacetalverbindung, eines Acetonitrils, eines Trimethylchlorsilans und einer radioaktiven Jodquelle als Rohmaterialien;
Mischen der Rohmaterialien, und
Durchführen einer Reaktion bei einer Reaktionstemperatur von 20-50°C für eine Reaktionsdauer von 10-40 Minuten, um das Reagens zum Testen des Reinigungsvermögens von radioaktivem Gas in einem Kernkraftwerk zu erhalten.

**2.** Verfahren zum Herstellen des Reagens zum Testen des Reinigungsvermögens von radioaktivem Gas in einem Kernkraftwerk nach Anspruch 1, **dadurch gekennzeichnet, dass** das Volumenverhältnis der Methylphosphatverbindung oder der Dimethylacetalverbindung, des Acetonitrils und des Trimethylchlorsilans 0,1-2 : 3-6 : 0,1-2 ist.

**3.** Verfahren zum Herstellen des Reagens zum Testen des Reinigungsvermögens von radioaktivem Gas in einem Kernkraftwerk nach Anspruch 2, **dadurch gekennzeichnet, dass** die radioaktive Jodquelle ein radioaktives Iodid ist, das eine Radioaktivität von 10-400 MBq hat.

**4.** Verfahren zum Herstellen des Reagens zum Testen des Reinigungsvermögens von radioaktivem Gas in einem Kernkraftwerk nach Anspruch 3, **dadurch gekennzeichnet, dass** das radioaktive Iodid radioaktives Kaliumiodid oder radioaktives Natriumiodid ist.

**5.** Verfahren zum Herstellen des Reagens zum Testen des Reinigungsvermögens von radioaktivem Gas in einem Kernkraftwerk nach Anspruch 4, **gekennzeichnet durch** Verwenden des radioaktiven Iodids, des Acetonitrils, der Methylphosphatverbindung und des Trimethylchlorsilans als Rohmaterialien, Mischen der Rohmaterialien und Durchführen der Reaktion unter Einblasen von Edelgas oder unter Rühren, wobei die Reaktionstemperatur 20-30°C und die Reaktionszeit 10-30 Minuten beträgt.

**6.** Verfahren zum Herstellen des Reagens zum Testen des Reinigungsvermögens von radioaktivem Gas in einem Kernkraftwerk nach Anspruch 4, **gekennzeichnet durch** Verwenden des radioaktiven Iodids, des Acetonitrils, der Dimethylacetalverbindung und des Trimethylchlorsilans als Rohmaterialien, Mischen der Rohmaterialien und Durchführen der Reaktion unter Einblasen von Edelgas oder unter Rühren, wobei die Reaktionstemperatur 40-50°C und die Reaktionszeit 20-40 Minuten beträgt.

**7.** Verfahren zum Herstellen des Reagens zum Testen des Reinigungsvermögens von radioaktivem Gas in einem Kernkraftwerk nach Anspruch 2, **dadurch gekennzeichnet, dass** die radioaktive Jodquelle hergestellt wird durch Mischen einer Pufferverteilungslösung mit der Wasserlösung von radioaktivem Iodid; die Radioaktivität der hergestellten radioaktiven Jodquelle 10-400 MBq beträgt; die Pufferverteilungslösung durch Mischen eines nicht radioaktiven Iodids mit einem Azeton hergestellt wird.

**8.** Verfahren zum Herstellen des Reagens zum Testen des Reinigungsvermögens von radioaktivem Gas in einem Kernkraftwerk nach Anspruch 7, **dadurch gekennzeichnet, dass** das radioaktive Iodid radioaktives Kaliumiodid oder radioaktives Natriumiodid ist, das nicht radioaktive Iodid nicht radioaktives Kaliumiodid oder nicht radioaktives Natriumiodid ist.

**9.** Verfahren zum Herstellen des Reagens zum Testen des Reinigungsvermögens von radioaktivem Gas in einem Kernkraftwerk nach Anspruch 8, **dadurch gekennzeichnet, dass** es die folgenden Schritte umfasst:

Schritt (1) Mischen des Acetonitrils und des Azetons, und dann Hinzufügen des nicht radioaktiven Iodids, um eine Mischlösung des Acetonitrils und der Pufferverteilungslösung zu erhalten, wobei das Verhältnis zwischen dem Acetonitril, dem nicht radioaktiven Iodid und der Pufferverteilungslösung 1-4 ml : 0,1-5 g : 0,5-2 ml beträgt;
Schritt (2) Hinzufügen der Wasserlösung von radioaktivem Iodid zu der Mischlösung von Schritt (1), um eine Mischung des Acetonitrils und der radioaktiven Jodquelle zu erhalten;
Schritt (3) Hinzufügen der Methylphosphatverbindung oder der Dimethylacetalverbindung und des Trimethylchlorsilans zu der Mischung des Acetonitrils und der radioaktiven Jodquelle von Schritt (2), Mischen der Rohmaterialien und Durchführen der Reaktion, um das Reagens zum Testen des Reinigungsvermögens von radioaktivem Gas in einem Kernkraftwerk zu erhalten, unter Einblasen von Edelgas oder unter Rühren, wobei die Reaktionstemperatur 20-50°C und die Reaktionszeit 10-40 Minuten beträgt.

**10.** Verfahren zum Herstellen des Reagens zum Testen des Reinigungsvermögens von radioaktivem Gas in einem Kernkraftwerk nach Anspruch 9, **dadurch gekennzeichnet, dass** in Schritt (3) die Methylphosphatverbindung und das Trimethylchlorsilan der Mischung der radioaktiven Jodquelle und des Acetonitrils hinzugefügt werden, sie gemischt werden, um einheitlich zu sein, und die Reaktion unter Einblasen von Edelgas oder unter Rühren durchgeführt wird, wobei die Reaktionstemperatur 20-30°C und die Reaktionszeit 10-30 Minuten beträgt.

**11.** Verfahren zum Herstellen des Reagens zum Testen des Reinigungsvermögens von radioaktivem Gas in einem Kernkraftwerk nach Anspruch 9, **dadurch gekennzeichnet, dass** in Schritt (3) die Dimethylacetalverbindung und das Trimethylchlorsilan der Mischung der radioaktiven Jodquelle und des Acetonitrils hinzugefügt werden, sie gemischt werden, um einheitlich zu sein, und die Reaktion unter Einblasen von Edelgas oder unter Rühren durchgeführt wird, wobei die Reaktionstemperatur 40-50°C und die Reaktionszeit 20-40 Minuten beträgt.

**12.** Verfahren zum Herstellen des Reagens zum Testen des Reinigungsvermögens von radioaktivem Gas in einem Kernkraftwerk nach einem der Ansprüche 1 bis 11, **dadurch gekennzeichnet, dass** die Methylphosphatverbindung $R_1PO(OCH_3)_2$ ist, wobei $R_1$ $PhClCH_2$, $CCl_3$, $NCCH_2$, MeCO, $MeOCOCH_2$, $EtOCOCH_2$, t-BuOCOCH2, $PhCH_2OCOCH_2$, EtOCO, $Et_2NCO$, MeCOOCHPh, $PhCOCH_2$, $MeOCH_2$, $(MeO)_2CHCH_2$ oder $(MeO)_2CH$ ist.

**13.** Verfahren zum Herstellen des Reagens zum Testen des Reinigungsvermögens von radioaktivem Gas in einem Kernkraftwerk nach einem der Ansprüche 1 bis 11, **dadurch gekennzeichnet, dass** die Dimethylacetalverbindung $R_2R_3C(OCH_3)_2$ ist, wobei $R_2$ Ph, $Me(CH_2)_5$ oder $(CH_2)_5$ und $R_3$ Me oder H ist.

**14.** Verfahren zum Herstellen des Reagens zum Testen des Reinigungsvermögens von radioaktivem Gas in einem Kernkraftwerk nach einem der Ansprüche 1 bis 11, **gekennzeichnet durch** Hinzufügen des Acetonitrils und des radioaktiven Kaliumiodids der Reihe nach in einen Reaktionsbehälter, dann Hinzufügen eines Ethyl-Dimethyl-Phosphonoacetats und des Trimethylchlorsilans in den Reaktionsbehälter und Durchführen der Reaktion unter Rühren, um das Reagens zu erhalten, wobei die Reaktionstemperatur 30°C und die Reaktionszeit 15 Minuten beträgt, und das Verhältnis zwischen dem Acetonitril, dem radioaktiven Kaliumiodid, dem Ethyl-Dimethyl-Phosphonoacetat und dem Trimethylchlorsilan 3-6 ml : 3-4 g : 0,1-2 ml : 0,1-2 ml ist.

**15.** Verfahren zum Herstellen des Reagens zum Testen des Reinigungsvermögens von radioaktivem Gas in einem Kernkraftwerk nach einem der Ansprüche 1 bis 11, **gekennzeichnet durch** Hinzufügen des Acetonitrils und des radioaktiven Kaliumiodids der Reihe nach in einen Reaktionsbehälter, dann Hinzufügen eines O-Chlorphenyl-Dimethyl-Phosphats und des Trimethylchlorsilans in den Reaktionsbehälter und Durchführen der Reaktion unter Rühren, um das Reagens zu erhalten, wobei die Reaktionstemperatur 25°C und die Reaktionszeit 10 Minuten beträgt, und das Verhältnis zwischen dem Acetonitril, dem radioaktiven Kaliumiodid, dem O-Chlorphenyl-Dimethyl-Phosphat und dem Trimethylchlorsilan 3-6 ml : 3-4 g : 0,1-2 ml : 0,1-2 ml ist.

**16.** Verfahren zum Herstellen des Reagens zum Testen des Reinigungsvermögens von radioaktivem Gas in einem Kernkraftwerk nach einem der Ansprüche 1 bis 11, **gekennzeichnet durch** Hinzufügen des Acetonitrils und des radioaktiven Kaliumiodids der Reihe nach in einen Reaktionsbehälter, dann Hinzufügen eines Benzaldehyd-Dimethylacetals und des Trimethylchlorsilans in den Reaktionsbehälter und Durchführen der Reaktion unter Rühren, um das Reagens zu erhalten, wobei die Reaktionstemperatur 40°C und die Reaktionszeit 20 Minuten beträgt, und das Verhältnis zwischen dem Acetonitril, dem radioaktiven Kaliumiodid, dem Benzaldehyd-Dimethylacetal und dem Trimethylchlorsilan 3-6 ml : 3-4 g : 0,1-2 ml : 0,1-2 ml ist.

**17.** Verfahren zum Herstellen des Reagens zum Testen des Reinigungsvermögens von radioaktivem Gas in einem Kernkraftwerk nach einem der Ansprüche 1 bis 11, **gekennzeichnet durch** Hinzufügen des Acetonitrils und des radioaktiven Kaliumiodids der Reihe nach in einen Reaktionsbehälter, dann Hinzufügen von $(CH_2)_5C(OCH_3)_2$ und des Trimethylchlorsilans in den Reaktionsbehälter und Durchführen der Reaktion unter Rühren, um das Reagens zu erhalten, wobei die Reaktionstemperatur 45°C und die Reaktionszeit 30 Minuten beträgt, und das Verhältnis zwischen dem Acetonitril, dem radioaktiven Kaliumiodid, dem $(CH_2)_5C(OCH_3)_2$ und dem Trimethylchlorsilan 3-6 ml : 3-4 g : 0,1-2 ml : 0,1-2 ml ist.

**18.** Iodidfiltertestausrüstung für ein Kernkraftwerk, die speziell dazu ausgelegt ist, ein Reagens zu verwenden, das hergestellt wird durch Verwenden einer Methylphosphatverbindung oder einer Dimethylacetalverbindung, eines Acetonitrils, eines Trimethylchlorsilans und einer radioaktiven Jodquelle als Rohmaterialien, Mischen der Rohmaterialien, und Durchführen einer Reaktion bei 20-50°C, wobei die Iodidfiltertestausrüstung einen negativen Druckkasten (2), einen Druckluftverteiler (4), der sich auf dem negativen Druckkasten (2) befindet, einen Methyliodidgenerator und eine pneumatische Steuereinheit aufweist, die sich in dem negativen Druckkasten (2) befindet; wobei der Methyliodidgenerator eine Druckluft-Einspritzpumpe (3), ein Rückschlagventil (5), eine Drossel (6), einen Generatorkörper (23), eine Hülsennadel (18), einen Reaktionsbehälter (17), ein Hebegestell für den Reaktionsbehälter (16) und einen Zylinder (13) zum Auf- und Abbewegen des Hebegestells aufweist; und wobei der Methyliodidgenerator mit einem Einlassrohr (204) verbunden ist, das mit der Atmosphäre außerhalb des negativen Druckkastens (2) verbunden ist; wobei das Einlassrohr (204) mit einer Innennadel der Hülsennadel (18) durch den Generatorkörper (23) verbunden ist und ein Lufteinlass der Druckluft-Einspritzpumpe (3) mit einem Raum zwischen der Innennadel und einer Außennadel der Hülsennadel (18) verbunden ist; wobei die pneumatische Steuereinheit ein Mikrodrucksignalventil (8), ein Druck-Zug-Umschaltventil (10), ein Zweipositions-Fünfwege-Einweg-Luftsteuer-Umschaltventil

(11), ein Einweg-Drosselventil (7) und ein Rückschlagventil (9) aufweist; **dadurch gekennzeichnet, dass** das Druck-Zug-Umschaltventil (10) ein pneumatisch zurückgesetztes ist und das Einlassrohr (204) mit einem nicht negativen Druck-Schalter (26) mit Zeitverzögerungsausgang und einem Rückschlagventil versehen ist.

**19.** Iodidfiltertestausrüstung für ein Kernkraftwerk nach Anspruch 18,
**dadurch gekennzeichnet, dass** der nicht negative Druck-Schalter (26) mit Zeitverzögerungsausgang mit dem pneumatisch zurückgesetzten Druck-Zug-Umschaltventil (10) verbunden ist, wobei der nicht negative Druck-Schalter (26) mit Zeitverzögerungsausgang Gas an das pneumatisch zurückgesetzte Druck-Zug-Umschaltventil (10) ausgibt, um das pneumatisch zurückgesetzte Druck-Zug-Umschaltventil (10) zu schließen.

**20.** Iodidfiltertestausrüstung für ein Kernkraftwerk nach Anspruch 19,
**dadurch gekennzeichnet, dass** das pneumatisch zurückgesetzte Druck-Zug-Umschaltventil (10) einen Druck-Zug-Umschaltventil-Zylinder (101) aufweist; wobei im Inneren des Druck-Zug-Umschaltventil-Zylinders (101) drei Kolben (102, 103, 104) in Reihe angeordnet sind und ein Griff (105), der mit den Kolben (102, 103, 104) verbunden ist, um die Kolben (102, 103, 104) zu ziehen oder zu drücken, außerhalb des Druck-Zug-Umschaltventil-Zylinders (101) angeordnet ist; fünf Poren (b, a, c, P', d), die eine erste Pore (b) bis fünfte Pore (d) aufweisen, sich in der Wand des Druck-Zug-Umschaltventil-Zylinders (101) befinden; beide Enden des Druck-Zug-Umschaltventil-Zylinders (101) jeweils mit einem ersten Druckausgleichsloch (P1) und einem zweiten Druckausgleichsloch (e) versehen sind, die verwendet werden, um den Innen- und Außendruck des Druck-Zug-Umschaltventil-Zylinders (101) während der Bewegung der Kolben (102, 103, 104) auszugleichen; wenn sich das pneumatisch zurückgesetzte Druck-Zug-Umschlagventil (10) durch Herausziehen des Griffs (105) im offenen Zustand befindet, ist eine zweite Pore (a) mit einer vierten Pore (P') verbunden, die erste Pore (b) ist mit einer dritten Pore (c) verbunden, und die fünfte Pore (d) wird durch den dritten Kolben (104) abgedichtet; wenn sich das pneumatisch zurückgesetzte Druck-Zug-Umschaltventil (10) durch Hineinschieben des Griffs (105) im geschlossenen Zustand befindet, ist die erste Pore (b) mit der vierten Pore (P') verbunden, die zweite Pore (a) ist mit der fünften Pore (d) verbunden, und die dritte Pore (c) wird durch den ersten Kolben (102) abgedichtet; der Kolben durch eine Zugstange (106) verbunden ist und das erste Druckausgleichsloch (P1) mit dem nicht negativen Druck-Schalter (26) mit Zeitverzögerungsausgang verbunden ist.

**21.** Iodidfiltertestausrüstung für ein Kernkraftwerk nach einem der Ansprüche 18 bis 20, **dadurch gekennzeichnet, dass** der nicht negative Druck-Schalter (26) mit Zeitverzögerungsausgang einen nicht negativen Druck-Schalter (26) aufweist, wobei ein Zeitverzögerungsschalter des Weiteren an einem Ausgang des nicht negativen Druck-Schalters (26) angeordnet ist, um den Ausgang eines nicht negativen Druck-Signals zu verzögern; wobei der nicht negative Druck-Schalter (26) einen Zylinderblock (260) aufweist, der mit drei Kolben (262, 263, 264) im Inneren ausgelegt ist; wobei die drei Kolben (262, 263, 264) im Abstand angeordnet sind und des Weiteren in einer integrierten Kolbengruppe ausgebildet sind, die mit Federn (216, 266) versehen ist, welche auf beiden Seiten dieselbe elastische Kraft haben; wobei der Zylinderblock (260) mit einer ersten Entlüftungsöffnung (Z), einer zweiten Entlüftungsöffnung (T) und einer dritten Entlüftungsöffnung (E) ausgelegt ist, wobei ein Ende des Zylinderblocks (260) mit einem Druckleitungsloch (W) versehen ist, das dazu betreibbar ist, Gas zum Bewegen der Kolbengruppe auszugeben; wobei das Druckleitungsloch (W) mit einem Innennadel-Lufteinlass an dem Generatorkörper (23) durch eine Leitung verbunden ist; wenn das Druckleitungsloch (W) nicht mit einem Gaseingang versehen wird oder mit einem positiven Druckeingang versehen wird, ist die zweite Entlüftungsöffnung (T) mit der dritten Entlüftungsöffnung (E) verbunden und der nicht negative Druck-Schalter (26) befindet sich im offenen Zustand; wenn das Druckleitungsloch (W) mit einem negativen Druckeingang versehen wird, ist das erste Durchgangsloch wegen der Bewegung der Kolbengruppe mit dem zweiten Durchgangsloch verbunden, und der nicht negative Druck-Schalter (26) befindet sich im geschlossenen Zustand.

**22.** Iodidfiltertestausrüstung für ein Kernkraftwerk nach einem der Ansprüche 18 bis 20, **dadurch gekennzeichnet, dass** die Hülsennadel (18) eine Innennadel und eine Außennadel aufweist, die zueinander konzentrisch sind, wobei die Innennadel von der Außennadel umgeben ist und der Abstand zwischen der Innennadel und der Außennadel 0,5 - 1,5 mm beträgt.

## Revendications

**1.** Procédé de préparation d'un réactif pour tester la capacité de purification de gaz radioactif dans une centrale nucléaire, **caractérisé par** la prise d'un composé phosphate de méthyle ou d'un composé diméthylacétal, d'un acétonitrile, d'un triméthylchlorosilane et d'une source d'iode radioactif en tant que matières premières ; le mélange desdites matières premières, et la réalisation d'une réaction à une température de réaction de 20 à 50 °C pendant

une durée de réaction de 10 à 40 min pour obtenir le réactif pour tester la capacité de purification de gaz radioactif dans une centrale nucléaire.

2. Procédé de préparation du réactif pour tester la capacité de purification de gaz radioactif dans une centrale nucléaire selon la revendication 1, **caractérisé en ce que** le rapport en volume dudit composé phosphate de méthyle ou dudit composé diméthylacétal, dudit acétonitrile et dudit triméthylchlorosilane est de 0,1 à 2:3 à 6:0,1 à 2.

3. Procédé de préparation du réactif pour tester la capacité de purification de gaz radioactif dans une centrale nucléaire selon la revendication 2, **caractérisé en ce que** ladite source d'iode radioactif est un iodure radioactif qui a une radioactivité de 10 à 400 MBq.

4. Procédé de préparation du réactif pour tester la capacité de purification de gaz radioactif dans une centrale nucléaire selon la revendication 3, **caractérisé en ce que** ledit iodure radioactif est l'iodure de potassium radioactif ou l'iodure de sodium radioactif.

5. Procédé de préparation du réactif pour tester la capacité de purification de gaz radioactif dans une centrale nucléaire selon la revendication 4, **caractérisé par** la prise dudit iodure radioactif, dudit acétonitrile, dudit composé phosphate de méthyle et dudit triméthylchlorosilane en tant que lesdites matières premières, le mélange desdites matières premières et la réalisation de la réaction sous soufflage de gaz inerte ou sous agitation ; dans lequel une température de réaction est de 20 à 30 °C et une durée de réaction est de 10 à 30 min.

6. Procédé de préparation du réactif pour tester la capacité de purification de gaz radioactif dans une centrale nucléaire selon la revendication 4, **caractérisé par** la prise dudit iodure radioactif, dudit acétonitrile, dudit composé diméthylacétal et dudit triméthylchlorosilane en tant que lesdites matières premières, le mélange desdites matières premières et la réalisation de la réaction sous soufflage de gaz inerte ou sous agitation ; dans lequel une température de réaction est de 40 à 50 °C et une durée de réaction est de 20 à 40 min.

7. Procédé de préparation du réactif pour tester la capacité de purification de gaz radioactif dans une centrale nucléaire selon la revendication 2, **caractérisé en ce que** ladite source d'iode radioactif est préparée par mélange d'une solution de distribution de tampon avec la solution aqueuse d'iodure radioactif ; la radioactivité de la source d'iode radioactif préparée est de 10 à 400 MBq ; la solution de distribution de tampon est préparée par mélange d'un iodure non radioactif avec une acétone.

8. Procédé de préparation du réactif pour tester la capacité de purification de gaz radioactif dans une centrale nucléaire selon la revendication 7, **caractérisé en ce que** ledit iodure radioactif est l'iodure de potassium radioactif ou l'iodure de sodium radioactif, ledit iodure non radioactif est l'iodure de potassium non radioactif ou l'iodure de sodium non radioactif.

9. Procédé de préparation du réactif pour tester la capacité de purification de gaz radioactif dans une centrale nucléaire selon la revendication 8, **caractérisé en ce que** comprenant les étapes suivantes :

   étape (1), mélange dudit acétonitrile et de ladite acétone, et ensuite ajout dudit iodure non radioactif pour obtenir une solution mixte dudit acétonitrile et de ladite solution de distribution de tampon, dans laquelle le rapport entre ledit acétonitrile, ledit iodure non radioactif et ladite solution de distribution de tampon est 1 à 4 ml:0,1 à 5 g:0,5 à 2 ml ;
   étape (2), ajout de la solution aqueuse d'iodure radioactif dans ladite solution mixte de l'étape (1) pour obtenir un mélange dudit acétonitrile et de ladite source d'iode radioactif ;
   étape (3), ajout dudit composé phosphate de méthyle ou dudit composé diméthylacétal et dudit triméthylchlorosilane dans ledit mélange dudit acétonitrile et de ladite source d'iode radioactif de l'étape (2), mélange desdites matières premières et réalisation de la réaction pour obtenir ledit réactif pour tester la capacité de purification de gaz radioactif dans la centrale nucléaire sous soufflage de gaz inerte ou sous agitation ; dans laquelle une température de réaction est 20 à 50 °C et une durée de réaction est 10 à 40 min.

10. Procédé de préparation du réactif pour tester la capacité de purification de gaz radioactif dans une centrale nucléaire selon la revendication 9, **caractérisé par** l'ajout, à ladite étape (3), dudit composé phosphate de méthyle et dudit triméthylchlorosilane dans ledit mélange de ladite source d'iode radioactif et dudit acétonitrile, leur mélange jusqu'à uniformité et la réalisation de la réaction sous soufflage de gaz inerte ou sous agitation ; dans lequel la température de réaction est 20 à 30 °C et la durée de réaction est 10 à 30 min.

**11.** Procédé de préparation du réactif pour tester la capacité de purification de gaz radioactif dans une centrale nucléaire selon la revendication 9, **caractérisé par** l'ajout, à ladite étape (3), dudit composé diméthylacétal et dudit triméthylchlorosilane dans ledit mélange de ladite source d'iode radioactif et dudit acétonitrile, leur mélange jusqu'à uniformité et la réalisation de la réaction sous soufflage de gaz inerte ou sous agitation ; dans lequel la température de réaction est 40 à 50 °C et la durée de réaction est 20 à 40 min.

**12.** Procédé de préparation du réactif pour tester la capacité de purification de gaz radioactif dans une centrale nucléaire selon l'une quelconque des revendications 1 à 11, **caractérisé en ce que** ledit composé phosphate de méthyle est $R_1PO(OCH_3)_2$, dans lequel $R_1$ est $PhClCH_2$, $CCl_3$, $NCCH_2$, MeCO, MeO-$COCH_2$, $EtOCOCH_2$, t-$BuOCOCH_2$, $PhCH_2OCOCH_2$, EtOCO, $Et_2NCO$, MeCOOCHPh, $PhCOCH_2$, $MeOCH_2$, $(MeO)_2CHCH_2$ ou $(MeO)_2CH$.

**13.** Procédé de préparation du réactif pour tester la capacité de purification de gaz radioactif dans une centrale nucléaire selon l'une quelconque des revendications 1 à 11, **caractérisé en ce que** ledit composé diméthylacétal est $R_2R_3C(OCH_3)_2$, dans lequel $R_2$ est Ph, $Me(CH_2)_5$ ou $(CH_2)_5$, et $R_3$ est Me ou H.

**14.** Procédé de préparation du réactif pour tester la capacité de purification de gaz radioactif dans une centrale nucléaire selon l'une quelconque des revendications 1 à 11, **caractérisé par** l'ajout dudit acétonitrile et dudit iodure de potassium radioactif dans une fiole de réaction en séquence, puis l'ajout d'un diméthylphosphonoacétate d'éthyle et dudit triméthylchlorosilane dans ladite fiole de réaction et la réalisation de la réaction sous agitation pour obtenir ledit réactif ; dans lequel la température de réaction est 30 °C, la durée de réaction est 15 min ; le rapport entre ledit acétonitrile, ledit iodure de potassium radioactif, ledit diméthylphosphonoacétate d'éthyle et ledit triméthylchlorosilane est 3 à 6 ml:3 à 4 g:0,1 à 2 ml:0,1 à 2 ml.

**15.** Procédé de préparation du réactif pour tester la capacité de purification de gaz radioactif dans une centrale nucléaire selon l'une quelconque des revendications 1 à 11, **caractérisé par** l'ajout dudit acétonitrile et dudit iodure de potassium radioactif dans une fiole de réaction en séquence, puis l'ajout d'un O-chlorophényl diméthyl phosphate et dudit triméthylchlorosilane dans ladite fiole de réaction et la réalisation de la réaction sous agitation pour obtenir ledit réactif ; dans lequel la température de réaction est 25 °C, la durée de réaction est 10 min ; le rapport entre ledit acétonitrile, ledit iodure de potassium radioactif, ledit O-chlorophényl diméthyl phosphate et ledit triméthylchlorosilane est 3 à 6 ml:3 à 4 g:0,1 à 2 ml:0,1 à 2 ml.

**16.** Procédé de préparation du réactif pour tester la capacité de purification de gaz radioactif dans une centrale nucléaire selon l'une quelconque des revendications 1 à 11, **caractérisé par** l'ajout dudit acétonitrile et dudit iodure de potassium radioactif dans une fiole de réaction en séquence, puis l'ajout d'un benzaldéhyde diméthylacétal et dudit triméthylchlorosilane dans ladite fiole de réaction et la réalisation de la réaction sous agitation pour obtenir ledit réactif; dans lequel la température de réaction est 40 °C, la durée de réaction est 20 min ; le rapport entre ledit acétonitrile, ledit iodure de potassium radioactif, ledit benzaldéhyde diméthylacétal et ledit triméthylchlorosilane est 3 à 6 ml:3 à 4 g: 0,1 à 2 ml:0,1 à 2 ml.

**17.** Procédé de préparation du réactif pour tester la capacité de purification de gaz radioactif dans une centrale nucléaire selon l'une quelconque des revendications 1 à 11, **caractérisé par** l'ajout dudit acétonitrile et dudit iodure de potassium radioactif dans une fiole de réaction en séquence, puis l'ajout d'un $(CH_2)_5C(OCH_3)_2$ et dudit triméthylchlorosilane dans ladite fiole de réaction et la réalisation de la réaction sous agitation pour obtenir ledit réactif ; dans lequel la température de réaction est 45 °C, la durée de réaction est 30 min ; le rapport entre ledit acétonitrile, ledit iodure de potassium radioactif, ledit $(CH_2)_5C(OCH_3)_2$ et ledit triméthylchlorosilane est 3 à 6 ml:3 à 4 g:0,1 à 2 ml:0,1 à 2 ml.

**18.** Équipement de test de filtre d'iodure pour une centrale nucléaire qui est spécialement adapté pour utiliser un réactif qui est préparé en prenant un composé phosphate de méthyle ou un composé diméthylacétal, un acétonitrile, un triméthylchlorosilane et une source d'iode radioactif en tant que matières premières ; en mélangeant lesdites matières premières, et en réalisant une réaction à 20 à 50 °C ; l'équipement de test de filtre d'iodure comprenant une boîte à pression négative (2), un distributeur d'air comprimé (4) situé sur la boîte à pression négative (2), un générateur d'iodure de méthyle et une unité de commande pneumatique située à l'intérieur de la boîte à pression négative (2) ; dans lequel le générateur d'iodure de méthyle comprend une pompe d'injection d'air comprimé (3), un clapet anti-retour (5), un régulateur par étranglement (6), un corps de générateur (23), une aiguille à manchon (18), une fiole de réaction (17), un support de levage pour fiole de réaction (16) et un cylindre (13) pour déplacer le support de levage vers le haut haut et vers le bas ; le générateur d'iodure de méthyle est relié à un tuyau d'admission (204) qui est relié à l'atmosphère à l'extérieur de ladite boîte à pression négative (2) ; ledit tuyau d'admission (204) est

relié à une aiguille interne de ladite aiguille à manchon (18) à travers ledit corps de générateur (23), et une entrée d'air de ladite pompe d'injection d'air comprimé (3) est reliée à un espace entre ladite aiguille interne et une aiguille externe de ladite aiguille à manchon (18) ; ladite unité de commande pneumatique comprend une vanne de signal de micro-pression (8), une vanne d'inversion à poussée-traction (10), une vanne d'inversion de commande d'air unidirectionnelle à deux positions à cinq voies (11), une vanne d'étranglement unidirectionnelle (7) et un clapet anti-retour (9) ; **caractérisé en ce que**, ladite vanne d'inversion à poussée-traction (10) est une vanne à réinitialisation pneumatique, et ledit tuyau d'admission (204) est pourvu d'un interrupteur à pression non négative (26) avec sortie temporisée et d'un clapet anti-retour (27).

**19.** Équipement de test de filtre d'iodure pour une centrale nucléaire selon la revendication 18, **caractérisé en ce que** ledit interrupteur à pression non négative (26) avec sortie temporisée se connecte à ladite vanne d'inversion à poussée-traction à réinitialisation pneumatique (10), dans lequel ledit interrupteur à pression non négative (26) avec sortie temporisée délivre le gaz à ladite vanne d'inversion à poussée-traction à réinitialisation pneumatique (10) pour fermer ladite vanne d'inversion à poussée-traction à réinitialisation pneumatique (10).

**20.** Équipement de test de filtre d'iodure pour une centrale nucléaire selon la revendication 19, **caractérisé en ce que** ladite vanne d'inversion à poussée-traction à réinitialisation pneumatique (10) comprend un cylindre de vanne d'inversion à poussée-traction (101) ; dans lequel trois pistons (102, 103, 104) sont disposés en série à l'intérieur dudit cylindre de vanne d'inversion à poussée-traction (101), et une poignée (105) reliée auxdits pistons (102, 103, 104) pour tirer ou pousser lesdits pistons (102, 103, 104) est disposée à l'extérieur dudit cylindre de vanne d'inversion à poussée-traction (101) ; cinq pores (b, a, c, P', d) incluant un premier pore (b) jusqu'à un cinquième pore (d) sont situés dans la paroi dudit cylindre de vanne d'inversion à poussée-traction (101) ; les deux extrémités dudit cylindre de vanne d'inversion à poussée-traction (101) sont respectivement pourvues d'un premier trou d'équilibrage de pression (P1) et d'un deuxième trou d'équilibrage de pression (e) utilisés pour équilibrer les pressions interne et externe dudit cylindre de vanne d'inversion à poussée-traction (101) pendant le mouvement desdits pistons (102, 103, 104) ; lorsque ladite vanne d'inversion à poussée-traction à réinitialisation pneumatique (10) est dans un état ouvert par traction de ladite poignée (105), un deuxième pore (a) est relié à un quatrième pore (P'), ledit premier pore (b) est relié à un troisième pore (c), et ledit cinquième pore (d) est scellé par le troisième piston (104) ; lorsque ladite vanne d'inversion à poussée-traction à réinitialisation pneumatique (10) est dans un état fermé par poussée de ladite poignée (105), ledit premier pore (b) est relié audit quatrième pore (P'), ledit deuxième pore (a) est relié audit cinquième pore (d), et ledit troisième pore (c) est scellé par ledit premier piston (102) ;
ledit piston est relié par une tige de traction (106), et ledit premier trou d'équilibrage de pression (P1) est relié audit interrupteur à pression non négative (26) avec sortie temporisée.

**21.** Équipement de test de filtre d'iodure pour une centrale nucléaire selon l'une quelconque des revendications 18 à 20, **caractérisé en ce que** ledit interrupteur à pression non négative (26) avec sortie temporisée comprend un interrupteur à pression non négative (26), dans lequel un interrupteur à temporisation est en outre disposé au niveau d'une sortie dudit interrupteur à pression non négative (26) pour retarder la sortie d'un signal de pression non négative ;
ledit interrupteur à pression non négative (26) comprend un bloc-cylindre (260) qui est disposé avec trois pistons à l'intérieur (262, 263, 264) ; lesdits trois pistons (262, 263, 264) sont disposés dans l'intervalle et formés en outre en un groupe de pistons intégré, qui est pourvu de ressorts (216, 266) ayant la même force élastique sur ses deux côtés ; ledit bloc-cylindre (260) est agencé avec une première ouverture d'aération (Z), une deuxième ouverture d'aération (T) et une troisième ouverture d'aération (E) ; une extrémité dudit bloc-cylindre (260) est pourvue d'un trou de conduction de pression (W) qui est utilisable pour la sortie de gaz afin de déplacer le groupe de pistons ; ledit trou de conduction de pression (W) est relié à une entrée d'air par aiguille interne sur ledit corps de générateur (23) à travers un pipeline ; lorsque ledit trou de conduction de pression (W) n'est pas pourvu d'une entrée de gaz ou pourvu d'une entrée de pression positive, ladite deuxième ouverture d'aération (T) est reliée à ladite troisième ouverture d'aération (E), et ledit interrupteur à pression non négative (26) est dans un état ouvert ; lorsque ledit trou de conduction de pression (W) est pourvu d'une entrée de pression négative, ledit premier trou traversant est relié audit deuxième trou traversant en raison du déplacement dudit groupe de pistons, et ledit interrupteur à pression non négative (26) est dans un état fermé.

**22.** Équipement de test de filtre d'iodure pour une centrale nucléaire selon l'une quelconque des revendications 18 à 20, **caractérisé en ce que** ladite aiguille à manchon (18) comprend une aiguille interne et une aiguille externe qui sont concentriques l'une par rapport à l'autre, dans lequel ladite aiguille interne est entourée par ladite aiguille externe ; ledit espace entre ladite aiguille interne et ladite aiguille externe est 0,5 à 1,5 mm.

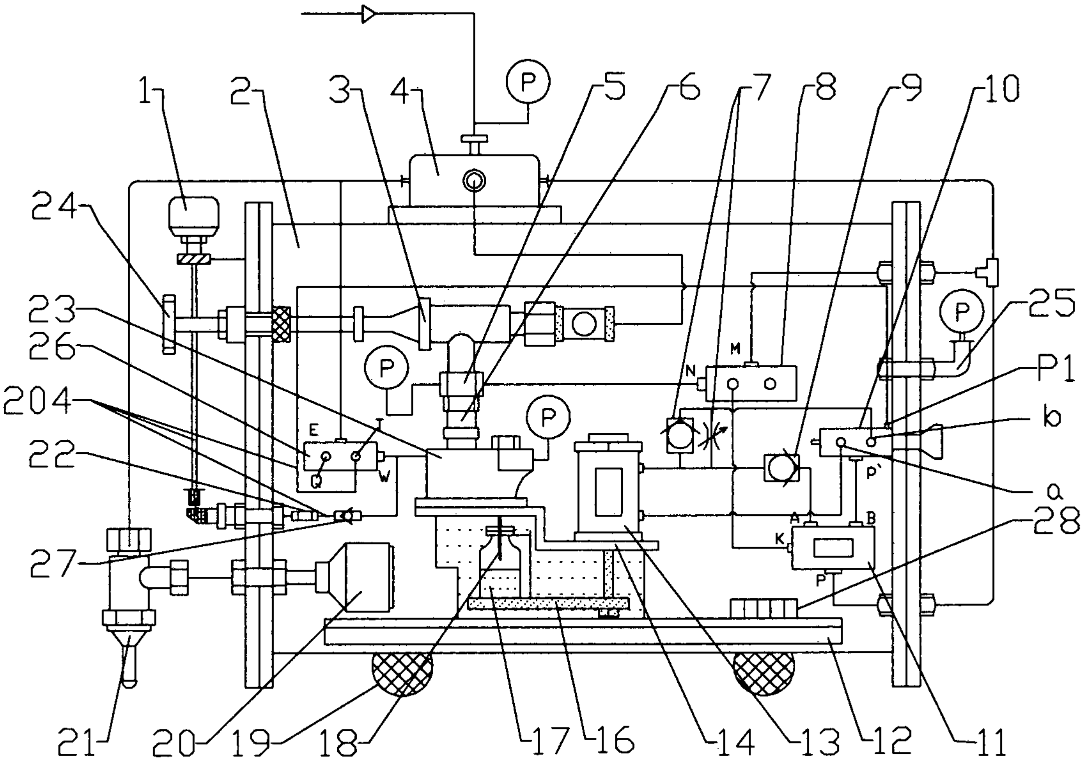


Figure 1

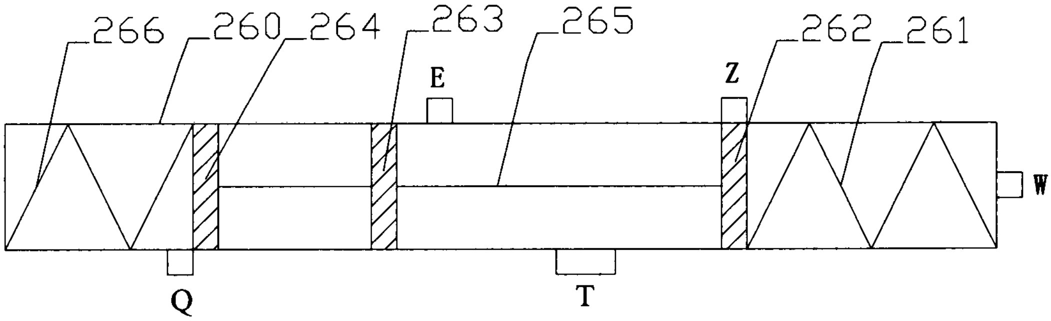


Figure 2

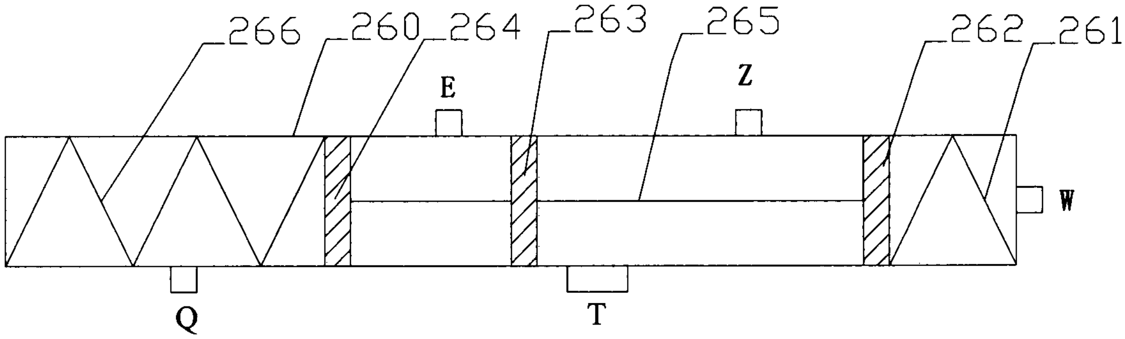


Figure 3

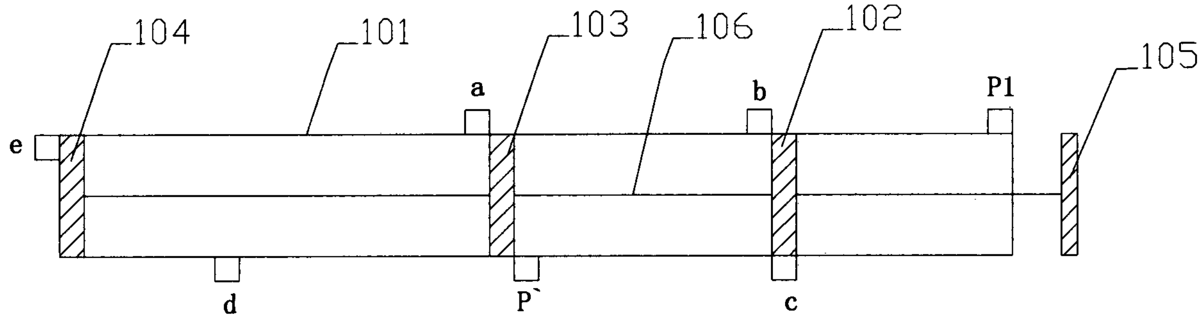


Figure 4

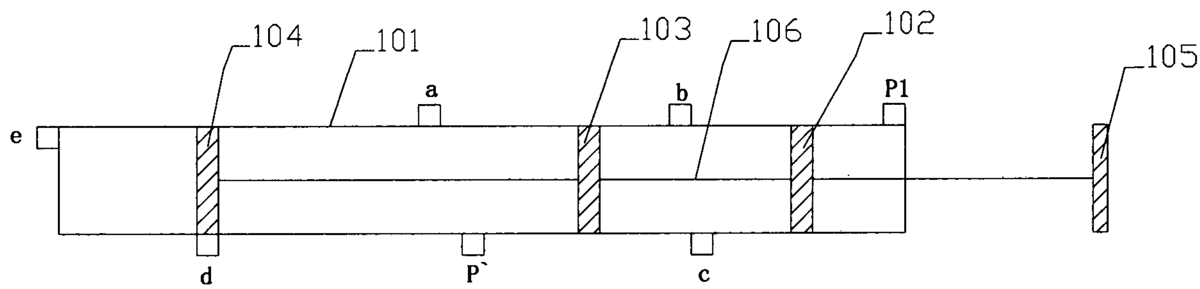


Figure 5

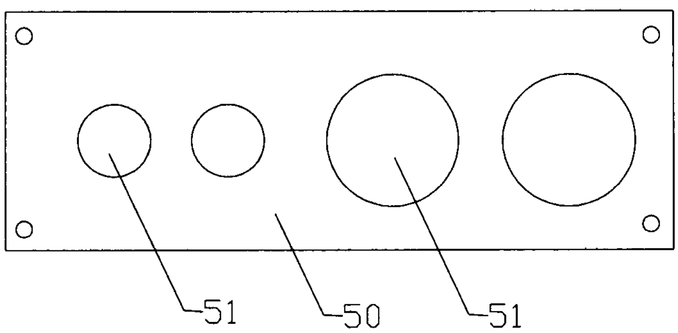


Figure 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 4016242 A
- FR 2553562 A1
- US 2006177944 A1